# EUROPEAN PATENT APPLICATION

(11) **EP 2 073 005 A1**
(43) Date of publication of application: **24.06.2009**
(21) Application number: 07150384.1
(22) Date of filing: 21.12.2007
(51) Int. Cl.: G01N 33/50

(54) **Method for in-vitro detecting pathogenic T helper cells and pharmaceutical compositions for treating autoimmune diseases**

(71) Applicant: Deutsches Rheuma-Forschungszentrum Berlin, 10117 Berlin (DE)
(72) Inventor: Radbruch, Andreas, 14552 Fresdorf (DE); Niesner, Uwe, 30853 Langenhagen (DE); Albrecht, Inka, 10437 Berlin (DE); Chang, Hyun-Dong, 10551 Berlin (DE); Stamm, Torsten, 53819 Neunkirchen-Seelscheid/Neunkirchen (DE); Grün, Joachim, 10117 Berlin (DE); Grützkau, Andreas, 10117 Berlin (DE)
(74) Representative: Ziebig, Marlene

(57) **Abstract**

The invention relates to a method for in-vitro detecting pathogenic T helper cells by incubating a sample of a body fluid or tissue taken from a mammal with substances specifically interacting with at least one gene product encoded by selected genes, which are differentially expressed compared to normal T helper cells, determining specific incubation products, correlating an amount of signal or change in signal with a concentration of the gene product in the sample, and detecting cell pathogenicity by comparing the concentration with another gene product concentration in a sample of non-pathogenic and/or pathogenic cells. Another object if the invention concerns a pharmaceutical composition for prophylaxis and therapy of chronic inflammatory. The invention also relates to a method for screening substances with the property to reduce the pathogenicity of T helper cells along with the symptoms of chronic inflammatory diseases.

## Description

The invention relates to a method for in-vitro detecting pathogenic T helper cells by incubating a sample of a body fluid or tissue taken from a mammal with substances specifically interacting with at least one gene product encoded by selected genes, which are differentially expressed compared to normal T helper cells, determining specific incubation products, correlating an amount of signal or change in signal with a concentration of the gene product in the sample, and detecting cell pathogenicity by comparing the concentration with another gene product concentration in a sample of non-pathogenic and/or pathogenic cells. Another object if the invention concerns a pharmaceutical composition for the prophylaxis and therapy of chronic inflammation. The invention also relates to a method for screening substances with the property to reduce the pathogenicity of T helper cells along with the symptoms of chronic inflammation.

Autoimmune diseases concern an exaggerated, chronic immune reaction directed against the body's tissue which is aberrantly assigned as deleterious target. Chronic inflammation finally results in the damage of the affected organs. Chronic inflammatory diseases include autoimmune hepatitis, chronic gastritis, diabetes mellitus type I, Morbus Crohn, Multiple Sclerosis, arthritis, psoriasis or rheumatism. Several therapeutic treatments are applied, which success has to be evaluated in each case. An abatement of symptoms and a reduction of acute attacks are achieved by cortisone. However, cortisone treatment is associated with a couple of adverse effects restricting its administration to a short period. Furthermore, the weakening of the immune system is known by immune suppressive agents. For instance, methotrexate is used to treat Multiple Sclerosis or rheumatism. Changes in hemogram, gastrointestinal dysfunction, nausea, diarrhea, tumor induction, alopecia and weight reduction arising from methotrexate administration are of disadvantage.

T helper 1 (Th1) lymphocytes control immune reactions and inflammation through the secretion of signal proteins. The potential to induce inflammation has been demonstrated by adoptive transfer of Th1 lymphocytes in murine models of Th1-associated inflammatory diseases such as diabetes, inflammatory bowel disease, and rheumatoid arthritis. In these models, the induction of inflammation in a particular tissue by Th1 lymphocytes is dependent on re-stimulation by their cognate antigen presented in that tissue. Induction of inflammation by Th1 cells is mediated by expression of the pro-inflammatory cytokines TNF-α and interferon-γ (IFN-γ), the latter being a hallmark of Th1 differentiation. IFN-γ also induces expression of the chemokine receptor CXCR3 and its ligands CXCL9, -10, and -11, attracting Th1 cells specifically to inflamed tissue. Th1 cells with the capacity to recall IFN-γ expression, i.e. Th1 memory cells, are detectable in chronically inflamed tissue.

The role of Th1 cells in chronification and maintenance of chronic inflammation is less clear. Initial studies aiming at a complete depletion of CD4+ T lymphocytes suggested a clinical benefit in respect of rheumatoid arthritis chronic and inflammatory arthritis, respectively, but studies had to be terminated due to the severe side effects of systemic depletion of Th lymphocytes (Choy et al. (1996) Arthritis Rheum 39:52-56; Emmrich et al. (1991) Agents Actions Suppl 32:165-170; Horneff et al. (1991) Arthritis Rheum 34:129-140). Skurkovich & Skurkovich (2006) Ernst Schering Res Found Workshop 1-27, as well as Hommes et al. (2006) Gut 55:1131-1137, showed that anti-IFN-γ therapy is beneficial in various Th1-associated inflammatory diseases. However, for IFN-γ, also a regulatory role has been demonstrated, based on the induction of inducible nitric oxide synthase (iNOS) and of IL-12 in antigen-presenting cells, in turn increasing the IL-10 synthesis of Th1 cells. Targeting of CD4 and CD3-expressing cells with non-depleting antibodies, and neutralization of Th-related effector cytokines have demonstrated clinical efficacy (Nepom (2002) Curr Opin Immunol 14:812-815, Panaccione et al. (2005) Curr Opin Pharmacol 5:566-572, Baumgart & Dignass (2004) Curr Pharm Des 10:4127-4147), but all of them impair protective as well as pathogenic T cell memory.

It has been recently suggested that pathogenic memory T cells are key players in chronic inflammation. Naïve Th lymphocytes are instructed by antigen and polarizing signals during their primary activation to express particular cytokines during this activation. Upon restimulation, the pre-activated Th cells recall the expression of those cytokines they had been instructed to express during their primary activation, with much faster kinetics of expression, and without the requirement of polarizing signals. As memory cells, they are stably imprinted for the expression of distinct cytokines, chemokines, adhesion molecules and other genes determining the function of Th cells. This memory is based on epigenetic modification of the cytokine genes and the presence of particular transcription factors. Genes specifically expressed in repeatedly activated Th cells may qualify as diagnostic and therapeutic targets. Consequently, the transcriptome analysis of repeatedly activated Th cells resulted in the identification of a marker for chronically activated Th1 cells.

Therefore, the technical problem forming the basis of the present invention is to provide further substances for the detection of pathogenic T helper, which makes a simple and fast determination of an instant autoimmune disease possible. It is another problem of the invention to provide a pharmaceutical composition allowing an effective application in diagnosis, prevention or therapy of chronic inflammation, especially such compositions that improve the therapeutic efficacy and minimize adverse effects. It is still another problem to provide a method for screening substances that effectively alter the expression pattern of differently expressed genes in pathogenic T helper cells.

The present invention solves the problem by providing a method for in-vitro detecting pathogenic T helper cells comprising the steps of incubating a sample of a body fluid or tissue taken from a mammal with substances specifically interacting with at least one gene product encoded by a gene that is selected from the group comprising the genes of Table 1 and Table 2, determining specific incubation products, correlating an amount of signal or change in signal with a concentration of the gene product in the sample, and detecting cell pathogenicity by comparing the concentration with another gene product concentration in a sample of non-pathogenic and/or pathogenic cells.

It has been surprisingly demonstrated by the inventors that the aforementioned group of 248 genes is correlated with pathogenicity of T helper cells. Consequently, the gene products of theses genes represent well suited targets for differentiating the stage of pathogenicity. The term "gene product" denotes molecules that are formed from the substrate of said genes by biochemical, chemical or physical reactions, such as DNA synthesis, transcription, splicing, translation, fragmentation or methylation. Preferred gene products of the invention are mRNA and proteins. The underlying genes are selected as result of a differential expression analysis. In this experimental approach, the transcriptional profiles of naive, once and repeatedly stimulated Th cells are compared using high-density DNA microarrays. The identified genes are not inevitably associated by function or location in their entity as presently known, but it is not excluded that such relations appear between single or more members of the group. Instead of that, all genes are characterized by a distinct difference to normal Th cells, which is exhibited by either up-regulation or repression by at least a factor of 2. The genes are already described in the state of the art by sequence and other features, but lacking a linkage to Th helper cells and the control of inflammation. The aforementioned genes may be named in another way, but are easily assigned by the accession number, which is generally accepted and fixed in numerous data bases, such as the GenBank, SwissProt and the like.

The linkage of T helper pathogenicity to distinct genes is utilized for the in-vitro detection of harmful Th cells, which promote inflammation, by means of a single marker. However, more than a single marker can be used for the utmost test reliability. That means the inventive principle underlying the present method comprises prospecting for a gene product that can be either detected on the genetic level or on the protein level. The gene product is chosen in respect of both its absolute and relative amount as well as the specificity for a certain T cell type. T helper cells can be distinguished into Th1 cells and Th2 cells, which fulfill different biological functions. Different differential expression pattern are recognized in Th1 and Th2 cells. Furthermore, the gene expression is widely restricted to a subset of memory cells. In an embodiment, the method of the invention relates to Th1 effector memory cells and/or Th2 effector memory cells.

In a preferred embodiment of the present invention, the gene product to be analyzed is encoded by a gene that is selected from the group comprising the genes of Table 1, which genes and the gene products thereof act as biomarkers for Th1 effector memory cells only.

In another preferred embodiment of the invention, the gene product to be analyzed is encoded by a gene that is selected from the group comprising the genes of Table 2, which genes and the gene products thereof act as biomarkers for Th2 effector memory cells only.

A sample of a body fluid or tissue is taken from a mammal to be tested. The sample is especially taken from a human or a mouse, preferably a human. The withdrawal of the body fluid or tissue sample follows good medical practice. In the present invention, the sample of body fluid preferably consists of blood, serum, plasma, saliva or urine. It is preferred to gather a tissue sample by biopsy, especially taken close to the location of ailment. The sample may be purified to remove disturbing substances, such as inhibitors for the formation of hydrogen bonds. Alternatively, the nucleic acid or protein material, respectively, can be concentrated in the sample. Downstream processing and/or concentrating are preferably performed by the methods of precipitation, dialysis, gel filtration, gel elution or chromatography, such as HPLC or ion exchange chromatography. It is recommended to combine several methods for better yields.

The human cell sample is stored, such as frozen, cultivated for a certain period or immediately incubated with substances that are specific for at least one gene product encoded by a gene that is selected from the aforementioned groups. The term "incubation" denotes the contacting of specific substances with the gene products for a distinct period, which depends on the kind of substance and/or target. The incubation procedure can be realized without a chemical conversion or may involve a biochemical reaction. Adding chemical solutions and/or applying physical procedures, e.g. impact of heat, can improve the accessibility of the gene products proteins in the sample. Specific incubation products are formed as result of the incubation.

The term "specific substances" as used herein comprises molecules with high affinity to at least one gene product encoded by the selected genes, in order to ensure a reliable binding. As described in detail in the further course of the specification, the term "specific substances" also comprise molecules with high affinity to the selected genes themselves, or a regulator protein or a gene product thereof, or a component of a signal transduction pathway comprising said gene or gene products thereof. Consequently, the specific interaction may be limited to the mere targeting, or the induction of alterations in cell function, or may even include both effects simultaneously. It shall be understood that variants, mutants, parts or homologous sequences having the same function, are included in the scope of definition as well as protection. The degree of alteration between the original sequence and its derivatives is inevitably limited by the requirement of gene product recognition within the structural context by means of the specific substances. Preferably, the homology amounts to at least 85 %. Possible alterations comprise deletion, insertion, substitution, modification and addition of at least one nucleotide or amino acid, respectively, or the fusion with another nucleic acid or protein. Preferably, the substances are mono-specific in order to guarantee an exclusive and directed interaction with the chosen single target.

The recognition of the target according to the invention can be realized by a specific interaction with substances on the primary, secondary and/or tertiary structure level of a nucleic acid sequence bearing the gene sequence or an amino acid sequence expressed by the gene. The coding function of genetic information favors the primary structure recognition, Contrary to that, the three-dimensional structure is mainly to be considered for protein recognition. In the context of the present invention, the term "recognition" - without being limited thereto - relates to any type of interaction between the specific substances and the target, particularly covalent or non-covalent binding or association, such as a covalent bond, hydrophobic/ hydrophilic interactions, van der Waals forces, ion pairs, hydrogen bonds, ligand-receptor interactions, interactions between epitope and antibody binding site, nucleotide base pairing, and the like. Such association may also encompass the presence of other molecules such as peptides, proteins or other nucleotide sequences.

The specific substances are composed of biological and/or chemical structures capable to interact with the target molecule in such a manner that makes a recognition, binding and interaction possible. In particular, the substances are selected from the group of nucleic acids, peptides, carbohydrates, polymers, small molecules having a molecular weight between 50 and 1.000 Da and proteins. The specific substances express a sufficient sensitivity and specificity in order to ensure a reliable detection.

The proteins or peptides are preferably selected from the group consisting of antibodies, cytokines, lipocalins, receptors, lectins, avidins, lipoproteins, glycoproteins, oligopeptides, peptide ligands and peptide hormones. More preferably, antibodies are used as specific substance. "Antibody" denotes a polypeptide essentially encoded by an immunoglobulin gene or fragments thereof. According to the invention, antibodies are present as intact immunoglobulins or a number of well-characterized fragments. Fragments are preferably selected from the group consisting of F_{ab} fragments, F_{c} fragments, single chain antibodies (scFv), variable regions, constant regions, H chain (V_{H}), and L chain (V_{L}), more preferably F_{ab} fragments and scFv. Fragments, such as F_{ab} fragments and F_{c} fragments, can be produced by cleavage using various peptidases. Furthermore, fragments can be engineered and recombinantly expressed, preferably scFv.

The term "nucleic acid" refers to a natural or synthetic polymer of single- or double-stranded DNA or RNA alternatively including synthetic, non-natural or modified nucleotides, which can be incorporated in DNA or RNA polymers. Each nucleotide consists of a sugar moiety, a phosphate moiety, and either a purine or pyrimidine residue. The nucleic acids are preferably single or double stranded DNA or RNA, primers, antisense oligonucleotides, ribozymes, DNA enzymes, aptamers and/or siRNA, or parts thereof. The nucleic acids can be optionally modified as phosphorothioate DNA, locked nucleic acid (LNA), peptide nucleic acid (PNA) or spiegelmer.

The specific substances can be labeled, in doing so the labeling depends on their inherent features and the detection method to be applied. For the detection of the specific incubation products, the applied methods depend on the specific incubation products to be monitored and are well known to the skilled artisan. Examples of suitable detection methods according to the present invention are fluorescence, luminescence, VIS coloring, radioactive emission, electrochemical processes, magnetism, or mass spectrometry.

Along with the detection, the concentration of the specific incubation products and the concentration of the gene product are determined. The signal intensity of the incubation products is correlated with the signal intensity of a calibration curve enabling the meter-reading of a matching concentration of the incubation product. Preferably, the calibration curve is based on the Lambert-Beer equation if using UV/VIS coloring or luminescence. The concentration of the gene product is subsequently calculated by considering the molar part of gene product within the product complex. Preferably, the molar ratio of specific substance and gene product is 1:1, which is present in antibody complexes for instance, so that the molar concentration of the incubation products corresponds to the molar concentration of the gene product.

Pathogenicity of Th cells is diagnosed by comparing the concentration of the gene product in the sample with known gene product concentration levels of either non-pathogenic cells and/or pathogenic cells. It shall be understood that the known concentrations are statistically proven, therefore representing a certain level or range, respectively. The direction and strength of gene expression have also been figured out by the differential expression analysis of the target genes of the invention such that either a distinct up-regulation or down-regulation with a certain factor has been recognized, which forms the basis of biomarker selection. Any measured concentration, which differs from the gene product concentration level of unstimulated Th cells, indicates an abnormality of the tested cell sample, whereas the cells are healthy at a gene product concentration which is comparable to the concentration level of unstimulated cells. It is preferred to measure concentration, which are higher than the gene product concentration level of unstimulated Th cells, for detecting pathogenicity. Contrary, a gene product concentration being in the range of the matching concentration of re-stimulated cells indicates a potential pathogenicity of the tested cells, whereas lower gene product concentrations than the aforementioned range confirm the absence Th cell pathogenicity.

Pathogenicity is influenced by the number of stimulating events, which the cells had experienced. Both, unstimulated cells, i.e. without any exposure to an antigen, and cells that are stimulated once by an antigen are regarded as not pathogenic. After a certain number of re-stimulations, a maximum is level is reached and remained stable thereafter. Surprisingly, it has been found that the gene product concentration is clearly correlated to the progress of Th cell pathogenicity. That means each concentration measured can be exactly assigned to a reference magnitude of gene product concentration on the calibration curve, the reference magnitude being in the range of a certain stage of pathogenicity.

It is an embodiment of the present invention that in the case of cell pathogenicity the gene product concentration exceeds at least twice the gene product concentration in a sample of unstimulated cells, preferably at least 15 times, more preferably at least 25 times, most preferably at least 40 times.

It is another embodiment of the present invention that in the case of cell pathogenicity the gene product concentration exceeds at least twice the gene product concentration in a sample of once antigen-stimulated cells, preferably at least 15 times, more preferably at least 25 times, most preferably at least 40 times.

Preferably, a gene is selected, which gene product may form incubation product on the cell surface or in the extracellular surrounding. Although receptor or membrane proteins are preferred, intracellular gene products, such as mRNA and intracellular proteins, can be used as target structure. A preferred encoding gene of the invention is the twist1 gene. Twist1 and the closely related twist2 (also known as dermo1) genes encode basic helix-loop-helix transcription factors involved in formation of mesoderm in Drosophila melanogaster, cranial neural tube and limb morphogenesis in mice, metastasis of tumor cells, control of apoptosis, and expression of cytokine genes in inflammation. Mice deficient for twist2 or haploinsufficient for both twist1 and twist2 succumb to severe systemic inflammation, demonstrating the central role and dose-dependency of Twist proteins for regulation of inflammation. Twist1 and twist2 are expressed by fibroblasts and macrophages, and expression is promoted by tumor necrosis factor-α (TNF-α) and type I interferons. The basic helix-loop-helix transcriptional repressor twist1, as an antagonist of NF-kB-dependent cytokine expression, is involved in the regulation of inflammation-induced immunopathology. Induction of twist1 in Th cells is dependent on nuclear factor kB (NF-kB), nuclear factor of activated T cells (NFAT), and interleukin 12 (IL-12) signaling via signal transducer and activator of transcription 4 (STAT4), and thus twist1 is specifically expressed by activated T helper 1 effector memory cells (Th1 EM cells). Expression of twist1 is transient, following T-cell receptor engagement and increments upon repeated stimulation of Th1 cells. Imprinting for enhanced twist1 expression marks repeatedly re-stimulated effector memory Th1 cells and thus the pathogenic memory Th cells of chronic inflammation. The cells isolated form the inflamed joint or chronically inflamed gut tissue of patients with ulcerative colitis or Crohn's disease, and synovial fluid of patients with spondyloarthopathies or rheumatoid arthritis are imprinted to express high levels of twist1, indicative of a history of repeated re-stimulation and an involvement in the pathogenesis of the disease by endogenous regulation of pro-inflammatory effector functions. Twist 1 reduces the functionality of Th1 cells by attenuating expression of interleukin 2 (IL-2), tumor necrosis factor-α (TNF-α), and the cytokines interferon-γ (IFN-γ), and ameliorates Th1-mediated immunopathology in delayed-type hypersensitivity and antigen-induced arthritis.

The method of the invention may comprise a further step, wherein such Th cells identified to be pathogenic are treated to be transferred into a non-pathogenic status, while remaining cell viability, or to be transferred into a status of reduced cell viability or even killed, which comes along with a diminished or abolished harmful impact of pathogenic cells. Thus, the present invention also relates to a method for reducing pathogenicity of T helper cells, by incubating a mammal cell sample comprising pathogenic Th cells with at least one substance being specific to at least one gene that is selected from the group comprising the genes of Table 1 and Table 2, or a regulator protein or a gene product thereof, or a component of a signal transduction pathway comprising said gene or gene products thereof. The specific interaction may comprise both the targeting of gene products to be detected as well as the functional effect. Alternatively, more than one substance can be used to fulfill a different function each. The in-vitro method is preferably applied to samples of mammals suffering from chronic inflammation. Testing of several specific substances makes the selection of that substance possible that is best suited for the treatment of the mammal subject. The in-vivo dose rate of the chosen substance is advantageously pre-adjusted to the pathogenicity of the specific cells with regard to their in-vitro data. Therefore, the therapeutic efficacy is remarkably enhanced. Preferably, a mono-specific substance is selected. More preferably, the substance is Twist1, or parts thereof, or the DNA encoding said substance. The ongoing teaching of the present specification concerning the use of Twist1 for the diagnosis, production of a medicament for the prophylactic or therapeutic treatment and/or monitoring is considered as valid and applicable without restrictions to the method for reducing pathogenicity of T helper cells if expedient.

It is another object of the invention to use substances specifically interacting with at least one gene product encoded by a gene that is selected from the group comprising the genes of Table 1 and Table 2 for detecting pathogenic T helper cells in-vitro. Ligand binding to gene products may be performed by substances selected from the group of nucleic acids, peptides, carbohydrates, polymers, small molecules having a molecular weight between 50 and 1.000 Da and proteins. Preferably, substances are used being specific to a gene product of the twist1 gene, such as twist1-mRNA or Twist1 protein for the diction of pathogenic T helper cells. The prior teaching of the present specification concerning the method for in-vitro detecting pathogenic T helper cells is considered as valid and applicable without restrictions to the in-vitro use of the substances for pathogenic T helper cell detection if expedient.

Further, the invention may be practiced as a kit comprising substances specifically interacting with at least one gene product encoded by a gene that is selected from the group comprising the genes of Table 1 and Table 2, particularly in order to perform the inventive method of detecting pathogenic T helper cells. The kit favorably comprises substances being specific to a gene product of the twist1 gene. The kit of the invention may include an article that comprises written instructions or directs the user to written instructions for how to practice the method of the invention. In an embodiment, the kit further comprises a reporter moiety or a reporter apparatus, preferably a fluorophore or a field-effect transistor. Additionally, the kit may comprise an extracting reagent for isolating an mRNA or a protein, for example, preferably containing the trancripted or translated twist1 gene. The prior teaching of the present specification concerning the detection method is considered as valid and applicable without restrictions to the kit if expedient.

Another object of the invention is a pharmaceutical composition comprising as active ingredient an effective amount of at least one protein encoded by a gene that is selected from the group comprising the genes of Table 1 and Table 2, or parts thereof, or the DNA encoding said ingredient, optionally together with pharmaceutically tolerable adjuvants. In a preferred embodiment of the present invention, the pharmaceutical composition comprises the *Twist1* protein. Alternatively, the pharmaceutical composition of the invention may comprise at least one substance specifically interacting with at least one gene that is selected from the group comprising the genes of Table 1 and Table 2, or a regulator protein or a gene product thereof, or a component of a signal transduction pathway comprising said genes or gene products thereof, optionally together with pharmaceutically tolerable adjuvants. Which kind of active ingredient is eventually used, i.e. either the protein or the specifically interacting substance, depends on the inherent function of the gene selected for implementing the inventive composition, and can be determined by those skilled in the art. Furthermore, a synergistic effect may be achieved by using more than one protein, or substance, respectively, and the compounds can be used either simultaneously or sequentially.

A "pharmaceutical composition" in the meaning of the invention is any agent in the field of medicine, which can be used in prophylaxis, diagnosis, therapy, follow-up or aftercare of patients who suffer from allergy, autoimmune diseases, gout, aberrance of the immune response, cancer and/or infection diseases in such a way that a pathogenic modification of their overall condition or of the condition of particular regions of the organism could establish at least temporarily.

The terms "effective amount" or "effective dose" or "dose" are interchangeably used herein and denote an amount of a pharmaceutical compound having a prophylactically or therapeutically relevant effect on a disease or pathological conditions. A prophylactic effect prevents the outbreak of a disease or even the infection with a pathogen after the infiltration of single representatives such that the subsequent propagation of the pathogen is strictly diminished, or it is even completely inactivated. A therapeutically relevant effect relieves to some extent one or more symptoms of a disease or returns to normal either partially or completely one or more physiological or biochemical parameters associated with or causative of the disease or pathological conditions. The respective dose or dosage range for administering the pharmaceutical composition according to the invention is sufficiently high in order to achieve the desired prophylactic or therapeutic effect of reducing symptoms of autoimmune diseases. It will be understood that the specific dose level, frequency and period of administration to any particular human will depend upon a variety of factors including the activity of the specific compound employed, the age, body weight, general health, sex, diet time of administration, route of administration, rate of excretion, drug combination and the severity of the specific therapy. Using well-known means and methods, the exact dose can be determined by one of skill in the art as a matter of routine experimentation.

The protein or the substance of the pharmaceutical composition can be conjugated to a chemotherapeutic, and/or fused or complexed with another molecule that promotes the directed transport to the destination, the incorporation into a pathogenic T helper cell, which is capable of differentially expressing said gene, and/or distribution within the pathogenic cells. It is especially preferred that the substance is conjugated to a chemotherapeutic if the substance does not exert any influence on the pathogenic Th cell by several reasons, such as the target is no receptor or regulator protein or component of a signal transduction pathway. While targeting a marker structure of pathogenic T helper cells by means of the substance, the chemotherapeutic moderates cell metabolism such that either differential gene expression is turned back or cell viability is shut down up to killing the cell. As a result of specific interaction on the surface, the construct of substance and chemotherapeutic may be subsequently incorporated into the desired pathogenic Th cells, which are clearly to be distinguished from non-pathogenic T helper cells and other cells types as well. The chemotherapeutic comprises a cytotoxin, a chemokine, a pro-apoptotic, an interferon, a radioactive moiety, or combinations thereof. Preferably, the chemotherapeutic moderates or alters nucleic acid metabolism, protein metabolism, cell division, DNA replication, purine biosynthesis, pyrimidine biosynthesis, amino acid biosynthesis, gene expression, mRNA processing, protein synthesis, apoptosis, or combinations thereof.

In particular, the pharmaceutical composition according to the invention comprises substances that are selected from the group of nucleic acids, peptides, carbohydrates, polymers, small molecules having a molecular weight between 50 and 1.000 Da and proteins. The substances are capable of discriminating between the background level if present and up-regulated concentrations of the gene product. That means, a minimum concentration of the gene product is required for binding and only concentrations exceeding the background threshold are measurable, or the gene product is exclusively expressed in pathogenic Th cells, the latter is preferred herein. The prior teaching of the present specification concerning the specific substances used in the method for in-vitro detecting pathogenic Th cells is considered as valid and applicable without restrictions to pharmaceutical compositions if these substances are expedient therein.

Preferably, small and/or compact nucleic acids are provided, which do not contact other structures within a tissue or organism, respectively, or which are not attacked by the prementioned ones, but specifically interact with the target molecule. The nucleic acids may be part of complexes or formulations consisting of lipids, carbohydrates, proteins or peptides, such as in the shape of a nano-capsule. More preferably, endogenous expression of genes can be silenced by RNA interference, which is based on dsRNA with 3' overlaps. Following the association of siRNA with specific proteins, a target RNA is recognized by the antisense siRNA strand and degraded by the intrinsic endonucleolytic activity of the ribonucleoprotein complex. The skilled artisan also knows theoretical models for the prediction of accessibility of mRNA regions. It is referred to the documents by Patzel et al. (Nucl. Acids. Res. 27, 4328-4334, 1999) as well as Scherr et al. (Nucl. Acids Res. 28, 2455-2461, 2000), which are incorporated as reference in the disclosure of the invention hereby. In doing so, the number of trial-and-error experiments is significantly reduced. Most preferably, cells are transfected with a small hairpin RNA (shRNA) of the sequence 5'-AAGCTGAGCAAGATTCAGACC-3'.

The composition of the invention is produced in a known way using common solid or liquid carriers, diluents and/or additives and usual adjuvants for pharmaceutical engineering and with an appropriate dosage depending on the intended mode of application. These pharmaceutically acceptable excipients comprise salts, buffers, fillers, chelating agents, antioxidants, solvents, bonding agents, lubricants, tablet coatings, flavor additives, flavors, preservatives and suspending agents. In the meaning of the invention, an "adjuvant" denotes every substance that enables, intensifies or modifies a specific immune response against the protein of the invention if administered simultaneously, contemporarily or sequentially. Known adjuvants for injection solutions are for example aluminum compositions, such as aluminum hydroxide or aluminum phosphate, saponins, such as QS21, muramyldipeptide or muramyltripeptide, proteins, such as gamma-interferon or TNF, M59, squalen or polyols. The amount of excipient material that is combined with the active substance to produce a single dosage form varies depending upon the host treated and the particular mode of administration.

The proteins are adapted in forms which are suitable for oral administration, such as tablets, film tablets, lozenges, capsules, pills, powders, solutions, dispersions, suspensions or depot forms thereof, for transdermal administration, such as solutions, suspensions, creams, ointments, gels, emulsions or band-aids, for parental administration, such as suppositories, and for intravenous infusion, subcutaneous injection or intramuscular administration, examples for the latter three are solutions and suspensions. The substances can also be adapted for topical, transmucosal, transurethal, vaginal, rectal or pulmonary administration in the appropriate formulations given above.

Depending upon the manner of introduction, the compounds may be formulated in a variety of ways. The concentration of therapeutically active ingredients in the formulation may vary from about 0.1 to 100 wt %. The solution may be administered alone or in combination with other treatments. In a preferred embodiment, the CEACAM proteins to be injected are in a water-soluble form, such as a pharmaceutically acceptable salt, which is meant to include both acid and base addition salts. The injection solution may also include one or more of the following: carrier proteins, such as serum albumin, buffers, stabilizing agents, coloring agents, and the like. Additives are well known in the art, and they are used in a variety of formulations.

Still another object of the invention is a protein, which is encoded by a gene that is selected from the group comprising the genes of Table 1 and Table 2, for the diagnosis, prophylactic or therapeutic treatment and/or monitoring of chronic inflammation. The protein of the invention also comprises variants, mutants, parts of the proteins or homologous sequences having the same function. A couple of methods are known to the skilled artisan to generate equivalent proteins, i.e. proteins that are analog in function to those of the inventive teaching. Therefore, the invention also contains the aforementioned modifications. For example, mutants can be generated by substitution, deletion, insertion, translocation, inversion and/or addition of at least a single amino acid. It is known that certain amino acids exhibit similar physicochemical characteristics making the substitution among each other possible. Variants of the protein can arise from modifications, such as alkylation, arylation or acetylation of at least a single amino acid, from incorporation of enantiomers and/or from fusion of the protein with a single or multiple amino acids, a peptide or a protein. It is preferred in the meaning of the invention that the protein is fused to a purification tag for affinity chromatography. Parts of the protein relates to a restriction to those regions that are sufficient for the expression of a specific function. All alterations are inevitably limited by the requirement of preserving the function. However, the parts of the protein can be very small due to the characterization of the binding site, which also triggers the signal cascade. In the meaning of the invention, it is to be clearly distinguished between protein parts of any size and homologous sequences which homology is related to the entire protein. Preferably, the homology between a natural protein and a derivative thereof, having the same features amounts to at least 60 %, more preferably 75 %, most preferably 90 %. Similarly, the homology is to be considered if the aforementioned part of any size is altered to a variant or mutant. In addition, several techniques are described in prior art to generate non-homologous peptides with the same function. The present teaching if solving the problem of the invention covers all peptide derivatives, which are developed on the basis of the present ingredients by such procedures.

The present invention also relates to DNA encoding said proteins of the invention for the diagnosis, prophylactic or therapeutic treatment and/or monitoring of autoimmune diseases. The prior teaching of the present specification concerning the amino acid sequences and derivatives thereof is valid and applicable without restrictions to the underlying DNA.

The protein and DNA of the invention are preferably used for the therapeutic treatment. A therapeutically relevant effect relieves to some extent one or more symptoms of an autoimmune disease, or returns to normality, either partially or completely, one or more physiological or biochemical parameters associated with or causative of the disease or pathological conditions. Monitoring is considered as a kind of treatment provided that the compounds are administered in distinct intervals, e.g. in order to booster the proliferation response and eradicate the symptoms of the disease completely. Either the identical compound or different compounds can be applied. In the meaning of the invention, prophylactic treatment is advisable if the subject possesses any preconditions for the outbreak of an autoimmune disease, such as a familial disposition, a genetic defect, or a previously passed disease.

The autoimmune diseases concerned by the invention are selected from the group comprising arthritis, autoimmune hepatitis, chronic gastritis, neurodermatitis, psoriasis, spondyloarthopathies, arthrosis, rheumatic diseases, rheumatoid arthritis, juvenile idiopathic arthritis, Crohn's disease, ulcerative colitis, diabetes, inflammatory bowel disease, Multiple Sclerosis, Systemic Lupus Erythematosus and/or allergic inflammations. Among the group, the inflammatory diseases and/or the allergic diseases are preferably addressed. Each of these disease subsets is clearly related to a defined type of pathogenic T helper cells. A protein, which is encoded by a gene that is selected from the group comprising the genes of Table 1, is exclusively used for the diagnosis, prophylactic or therapeutic treatment and/or monitoring of inflammatory diseases, preferably spondyloarthopathies, arthrosis, rheumatic diseases, rheumatoid arthritis, juvenile idiopathic arthritis, Crohn's disease, ulcerative colitis, diabetes, inflammatory bowel disease, Multiple Sclerosis and/or Systemic Lupus Erythematosus. A protein, which is encoded by a gene that is selected from the group comprising the genes of Table 2, is exclusively used for the diagnosis, prophylactic or therapeutic treatment and/or monitoring of allergic diseases, preferably allergic inflammations.

In a preferred embodiment of the invention, the Twist1 protein concerns the diagnosis, prophylactic or therapeutic treatment and/or monitoring of inflammatory diseases, preferably spondyloarthopathies, arthrosis, rheumatic diseases, rheumatoid arthritis, juvenile idiopathic arthritis, Crohn's disease, ulcerative colitis, diabetes, inflammatory bowel disease, Multiple Sclerosis and/or Systemic Lupus Erythematosus. The key role of twist 1 and Twist1, respectively, for the self-limitation of Th1 cells is evident from the analysis of models of inflammation. Ectopic overexpression of twist1 in Th1 cells, yet having a low endogenous expression level, drastically reduces their pathogenic contribution to delayed-type hypersensitivity. The fact that Th cells isolated from inflamed tissue of patients with chronic inflammatory gastrointestinal or rheumatic diseases show a dramatic up-regulation of twist1 expression, can be utilized by inducing or artificially supplying Twist1, e.g. administering an effective amount of Twist1 itself.

The invention also teaches a substance, which specifically interacts with at least one gene that is selected from the group comprising the genes of Table 1 and Table 2, or a regulator protein or a gene product thereof, or a component of a signal transduction pathway comprising said gene or gene products thereof, for the diagnosis, prophylactic or therapeutic treatment and/or monitoring of autoimmune diseases. Such substances directed against the selected genes of the invention, derivatives or associates thereof alter the functional effects triggered by them, and their interception modulates the immune response and may eliminate the accumulation of T cells within a center of inflammation. For example, an over-reaction of the immune system in a sick knee, which is full of blood or water due to a trauma or gout, respectively, is diminished or even completely prevented. The teaching of the present specification concerning the aforementioned clinical pictures in the context of using proteins for the diagnosis, prophylactic or therapeutic treatment and/or monitoring of autoimmune diseases is valid and applicable without restrictions to the substances if expedient, wherein the different approach of the compounds is recognized be the skilled artisan.

The invention also relates to the use of proteins encoded by a gene that is selected from the group comprising the genes of Table 1 and Table 2, or substances specifically interacting with said genes, or a regulator protein or a gene product thereof, or a component of a signal transduction pathway comprising said genes or gene products thereof, for the diagnosis, production of a medicament for the prophylactic or therapeutic treatment and/or monitoring of autoimmune diseases. The compounds, i.e. proteins or substances, can be either administered to prevent the initiation of autoimmune diseases of am mammal, preferably a human individual, and the resulting trouble in advance, or to treat the arising and continuing symptoms by tackling the molecular reasons. In an embodiment of the present invention, mono-specific compounds, preferably mono-specific substances, are used for the diagnosis, production of a medicament for the prophylactic or therapeutic treatment and/or monitoring of autoimmune diseases. The term "mono-specific" denotes a mode of binding which is characterized by the exclusive recognition of a single target. The mono-specific substances used in the present invention preferably recognize twist1 or any derivative thereof. The receptor/ligand-interaction is characterized by high affinity, high selectivity and minimal or even lacking cross-reactivity to other target molecules to exclude unhealthy and harmful impacts to the treated subject.

It is another object of the invention to provide a method for treating autoimmune diseases, wherein an effective amount of at least one substance specifically interacting with at least one gene that is selected from the group comprising the genes of Table 1 and Table 2, or a regulator protein or a gene product thereof, or a component of a signal transduction pathway comprising said genes or gene products thereof, is administered to a mammal in need of such treatment. The prior teaching of the invention and its embodiments is valid and applicable without restrictions to the method of treatment if expedient.

In addition, object of the invention is a method for screening substances, which reduce pathogenicity of T helper cells, comprising the steps of providing a sample of pathogenic T helper cells, which are capable of differentially expressing a gene that is selected from the group comprising the genes of Table 1 and Table 2, in comparison with non-pathogenic T helper cells, dividing the sample into portions, incubating at least one portion with substances to be screened, comparing the expression pattern and/or cell viability in the portion with another portion that is not incubated with the substances, and detecting the specific binding of substances to said genes or regulatory associated genes or regulator proteins or gene products thereof, or a component of a signal transduction pathway comprising said genes or regulatory associated genes or gene products thereof. The basic principles of such a screening method are described in detail in EP 1 780 220 A1, which is incorporated as reference in the disclosure of the invention hereby.

Briefly, the inventive method makes the identification and analysis of substances possible, which exert an influence on the signal cascade via the selected genes of the invention. The cell sample refers to primary cells or genetically engineered cells. The latter are capable of expressing these genes by transfection with appropriate vectors harboring them or parts thereof. Preferably, the recombinant cells are of eukaryotic origin. In an embodiment of the screening method, the pathogenic cells are provided by re-stimulating with an antigen, and compared to non-pathogenic cells not being stimulated or less stimulated by an antigen.

The cell sample is divided into multiple portions. At least two portions are provided; one is used for screening while the other one serves as negative control. Preferably, the number of portions for screening exceeds the number of control portions. Usually, numerous portions are subjected to a high-throughput screening.

The substances to be screened in the inventive method are not restricted anyway. In an embodiment of the invention, the substances are selected from the group of nucleic acids, peptides, carbohydrates, polymers, small molecules having a molecular weight between 50 and 1.000 Da, and proteins, preferably antibodies, cytokines and lipocalins. These substances are often available in libraries. It is preferred to incubate a single compound within a distinct portion of the cell sample. However, it is also possible to investigate the cooperative effect of substances by incubating at least two substances within one portion. A further portion of cells is simultaneously incubated in the absence of the substances. The incubation process of cells depends on various parameters, e.g. the cell type and the sensitivity of detection, which optimization follows routine procedures known to those skilled in the art.

The identification of effective substances in the meaning of the invention is indirectly performed by determining the expression patterns, which are altered, preferably moderated, and/or the cell viability, which is moderated, preferably apoptotic. The determination is performed at a specified moment and correlated to the signal strength at the beginning of the experiment and the negative control. Suitable tests are known to those skilled in the art or can be easily designed as a matter of routine.

Among those substances being revealed to reduce pathogenicity of Th cells each or some representatives are selected for further analysis. Preferably, the substances showing the greatest discrepancy to the control are chosen. They are analyzed for specificity to exclude another signal transduction, which is not initiated the binding to the genes of the invention and associated molecules thereof, and additionally tested for such a cross-reactivity in order to prevent adverse reactions or other effects by linked pathways if simultaneous docking to further receptor structures occur. Several methods are known in the field of the art for detecting specific and/or mono-specific binding, such as gel shift experiments, Biacore measurements, X-ray structure analysis, competitive binding studies, and the like. In a preferred embodiment of the screening method, the mono-specific binding of substances to the target structures of the invention is detected.

In another embodiment of the screening method, at least two subjects of a non-human organism suffering of an autoimmune disease are provided as sample, a subset of them the substances are administered, and the expression pattern and/or cell viability are indirectly compared via the symptoms of the disease in subjects to which substances have been administered and subjects to which no substances have been administered. The non-human organism is preferably a mammal, more preferably species such as mice or rats that may be genetically modified. It is possible to contact mice or rats, for example, with the substance candidates by injection, infusion, oral or rectal intake. It is preferred to incubate a single substance within a distinct portion of the non-human organisms.

In the scope of the present invention, a method for in-vitro detecting pathogenic T helper cells, which applies substances specifically interacting with at least one gene product encoded by a gene that is selected from the group comprising the genes of Table 1 and Table 2, is provided for the first time. The present invention teaches genes that are associated with pathogenicity of Th cells, and implies that the genes are involved in the pathogenic Th cell-induced aggravation of certain autoimmune diseases. Analysis of the differential expressed genes is very suitable for large-scale screening tests. In doing so, individuals are identified with an elevated risk for initiating or continuing autoimmune diseases. The detection method as well as arising diagnostic method of the invention can be performed in a simple and fast manner. In addition, the appropriate kit is cost-efficiently produced. The characterization of 248 genes critically involved in the persistence of Th cells in chronic or allergic inflammations also resulted in the provision of pharmaceutical compositions for the diagnosis, prophylactic or therapeutic treatment and/or monitoring of such human chronic inflammatory diseases. Their use is a promising, novel approach for a broad spectrum of therapies causing a direct and immediate reduction of symptoms. T cells, which trigger the autoimmune impulse, are advantageously influenced as pro-inflammatory imprinting is reversed and anti-inflammatory cytokine gene expression is induced to terminate inflammation. In chronic inflammatory diseases, particularly, twist 1 and the genes controlled by it are qualified as biomarkers for pathogenic effector memory cells. Targeting of twist1-expressing Th cells is highly specific for the pathogenic memory driving chronic inflammation. All proteins and substances are characterized by a high affinity, specificity and stability; low manufacturing costs and convenient handling. These features form the basis for a reproducible action, wherein the lack of cross-reactivity is included, and for a reliable and safe interaction with their matching target structures.

It is to be understood that this invention is not limited to the particular methods, pharmaceutical compositions, kit or uses described herein, as such matter may, of course, vary. It is also to be understood that the terminology used herein is for the purpose of describing particular embodiments only and is not intended to limit the scope of the present invention, which is only defined by the appended claims. As used herein, including the appended claims, singular forms of words such as "a," "an," and "the" include their corresponding plural referents unless the context clearly dictates otherwise. Thus, e.g., reference to "a substance" includes one or more different substances and reference to "a method" includes reference to equivalent steps and methods known to a person of ordinary skill in the art, and so forth. Unless otherwise defined, all technical and scientific terms used herein have the same meaning as commonly understood by a person of ordinary skill in the art to which this invention belongs.

Although methods and materials similar or equivalent to those described herein can be used in the practice or testing of the present invention, suitable examples are described below. The following examples are provided by way of illustration and not by way of limitation. Within the examples, standard reagents and buffers that are free from contaminating activities (whenever practical) are used.

### Mice and reagents

MRLlpr/Ipr, BALB/c, STAT4-deficient mice (Stat4t^{m1Gr}), and OVA-TCR^{tg/tg} DO11.10 mice were purchased from The Jackson Laboratory or were bred under specific pathogen-free conditions in our animal facility. All animal experiments were performed in accordance with institutional, state, and federal guidelines. Reagents were purchased from Sigma unless stated otherwise. BTP1 was synthesized by M. Paetzel (Humboldt University of Berlin, Germany), Cyclosporin A was purchased from Calbiochem.

### Patients

Endoscopic mucosal and surgical mucosal specimens were obtained from ulcerative colitis (UC, n = 9) and Crohn's disease (CD, n = 7) patients. UC and CD were diagnosed according to established clinical, endoscopic, radiologic and pathologic criteria. All UC and CD patients displayed moderately to severely active disease according to the Truelove and Witts Severity Index and the Harvey Bradshaw Severity Index, respectively. Control samples were obtained from patients (n = 4) undergoing colonectomy due to colon cancer. Mucosal control specimens used in the study were from the macroscopically noninvolved tissue distant to any detectable lesion. Synovial fluid was obtained from patients suffering from rheumatic diseases who had active synovitis with effusion. Patients with rheumatoid arthritis (RA, n = 4) fulfilled the American College of Rheumatology (ACR) 1987 classification criteria for RA, patients with ankylosing spondylitis (AS, n = 5) fulfilled the modified New York criteria (1984), and patients with psoriatic arthritis (PsA, n=3), reactive arthritis (ReA, n = 3) or undifferentiated spondyloarthritis (undiff. SpA, n = 2) fulfilled the European Spondyloarthropathy Study Group (ESSG) criteria for SpA. Clinical characteristics of the patients are listed in Table S2. All experiments were approved by the local ethics committee, and all patients gave informed consent.

### Isolation of human lymphocytes

Peripheral blood mononuclear cells (PBMC) from buffy coats from healthy donors and synovial fluid mononuclear cells were isolated by density gradient centrifugation (Lymphocyte separation medium, PAA). Pooled intraepithelial leukocytes (IEL) and lamina propria leukocytes (LPL) were obtained from mucosal specimens by treatment with collagenase type IV, followed by passage through a sieve. Mononuclear cells were collected from the 40% - 70% interphase of a discontinuous percoll (Pharmacia) gradient.

### Magnetic cell sorting

Anti-human CD4, anti-mouse CD4 and CD62L MACS MicroBeads were obtained from Miltenyi Biotec and were used according to the manufacturer's protocol.

### Flow cytometry

The following antibodies directed against murine antigens were either purified from hybridoma supernatants and conjugated in house or purchased as indicated: anti-CD3 (145-2C11), anti-CD4 (GK1.5), anti-CD8 (53-6.7), anti-CD11c (N418), anti-CD44 (IM7), anti-CD62L (MEL14), anti-DO11.10 OVA-TCR (KJ1.26), anti-B220 (RA3.6B2), anti-IL2 (JES6-5H4, Caltag), anti-IL-4 (11B11, BD Biosciences), anti-IFN-γ (AN18.17.24), anti-TNF-α (MP6-XT22, Caltag). Antibodies recognizing human antigens were obtained from BD Biosciences unless stated otherwise: anti-CD3 (OKT3, in house conjugate), anti-CD4 (TT1, in house conjugate), anti-CD27 (L128), anti-CD45RA (HI100), anti-CRTh2 (BM16), anti-CCR5 (2D7/CCR5). Cytometric analysis was performed with FACSCalibur using CellQuest (BD Biosciences) and FCS Express (De Novo) software. Cells were separated by fluorescence-activated cell sorting (FACSAria, FACSDiVa, BD Biosciences).

### Cell culture

Lymphocytes were cultured in complete RPMI1640 (Invitrogen) supplemented with 10% heat-inactivated fetal calf serum, 100 units/ml penicillin, 0.1 mg/ml streptomycin and 10 µM β-mercaptoethanol. Naïve CD4+CD62L+ lymphocytes from 6-8 w old DO11.10 mice were isolated as described. Irradiated (30 Gy) BALB/c splenocytes were used as APCs at a ratio of 5:1. The cognate peptide ova₃₂₃₋₃₃₉ (R. Volkmer-Engert, Humboldt University of Berlin, Germany) was added at 0.5 µM. Alternatively, plates were coated with 3 µg/ml anti-CD3 (145-2C11) in PBS, and CD4+ cells were plated at a density of 2x106 cells/ml in medium plus 1µg/ml soluble anti-CD28 (37.51). For Th1 differentiation, cells were stimulated in the presence of 5 ng/ml recombinant murine IL-12 (R&D Systems) and 5 µg/ml anti-IL-4 antibody (11B11). For Th2 differentiation, cells were stimulated in the presence of murine IL-4 (100 ng/ml, culture supernatant of HEK293T cells transfected with an expression plasmid, encoding murine IL-4), 5 µg/ml anti-IL-12 antibody (C17.8.6) and anti-IFN-γ antibody (AN 18.17.24). Dead cells were removed by Ficoll-Histopaque separation. Every 6 d viable Th cells were harvested and restimulated under the original conditions, except that 10 ng/ml murine IL-2 (R&D Systems) was added.

### Mitogenic re-stimulation and intracellular cytokine staining

Cells were re-stimulated with 10 ng/ml PMA, and 1 µg/ml ionomycin. Alternatively, cells were re-stimulated with plate-bound anti-CD3 (10µg/ml), and soluble anti-CD28 (1µg/ml). For re-stimulation of murine T cells, 10ng/ml IL-2 was added. For intracellular staining of cytokines, T cells were stimulated for 2 h with PMA/ionomycin and additional 3 h with 5 µg/ml of brefeldin A to block the secretion of cytokines. Cells were fixed with 2% formaldehyde in PBS for 15 min at room temperature and permeabilized with 0,5% w/v saponin.

### Chromatin Immunoprecipitation Assay (ChIP)

Cells were fixed with 2mM dithiobis (succinimidyl propionate) (DSP) for 30 min at room temperature, washed with PBS and fixed with 1% formaldehyde for 10 min. The chromatin was sheared to 200-1000 bp of length by sonification with five pulses of 10 s at 30% power (Bandelin, Berlin, Germany). The sheared chromatin was pre-cleared by incubation with Protein A MACS MicroBeads (Miltenyi Biotec) and incubated with anti-NFAT1 (AB1-209, ImmunoGlobe Antikoerpertechnik) or with anti-p65 (C-20, Santa Cruz) for 2 h at 4°C followed by incubation with Protein A microbeads for 30 min. Washing steps were performed on µ-columns (Miltenyi Biotec). Washing was performed sequentially with high salt, low salt, LiCl and TE buffer. Chromatin precipitate was eluted with 1% w/v SDS, 0.1 M NaHCO3 and 0,1% v/v β-Mercaptoethanol. Cross-links were reversed by incubation at 65°C overnight in the presence of 0.2 M NaCl, and the DNA was purified with Nucleospin Extract II (Macherey-Nagel). The relative amount of DNA was calculated with E^{ΔCp (input - immunoprecipitate)}. The following primers were used to amplify the proximal twist1 promoter: (-150 forward) 5'-GGGCTGGAAAGAGGAAACTT-3'; (+4 reverse) 5'-CGCGAGGTGTCTGAGAGTT-3'.

### Retroviral Infection

Retroviral stocks were obtained by calcium phosphate co-transfection of HEK293 cells with the retrovirus packaging plasmids pECO and pCGP. The medium was replaced after 4 h, and viral supernatants were collected 48 h later. Th cells were infected 40 h after activation by 60 min centrifugation at 700g at 30°C with viral supernatant and 8µg/ml polybrene, followed by 1 h at 37°C, and replacement of the viral supernatant with the former culture supernatant.

### Th1-mediated DTH model

The ova-specific DTh model was performed as previously described. Briefly, 5x10⁵ DO11.10 Th1 cells were injected i.v. into BALB/c mice, and 24 h later the DTh response was induced by s.c. injection of 250 ng ova₃₂₃₋₃₃₉/IFA into the left footpad. PBS/IFA, injected in the right footpad, served as a control. The footpad thickness was measured using an Oditest micrometer gauge (Kroeplin Laengenmesstechnik). The footpad thickness measured before the injection of the antigen was subtracted from the footpad thickness measured during the DTH response.

### Antigen-induced arthritis

2x10⁶ 2-w-old GFP+ Th1 cells were transferred i.v. into naïve SCID mice. 1 d later arthritis was induced by intraarticular injection of 60 µg cationised ovalbumin into one knee joint. The contralateral knee joint was left untreated. 3 weeks after disease induction, mice were sacrificed and knee joints were fixed in 10% formaldehyde, decalcified in saturated EDTA-solution and embedded in paraffin. Knee joint sections were stained with hematoxilin/eosin and scored for exudates, granulocyte infiltration, hyperplasia, fibroblast proliferation/mononuclear cell infiltration, periarticular mononuclear cell infiltration (each scoring 0-3), bone/cartilage destruction (scoring 0-4) and additional score of 1 for visible fibrin deposition and periarticular granulocyte infiltration, resulting in a maximum score of 21.

### Luciferase reporter assay

To monitor NF-κB activity in murine Th cells the pNFkB-Luc vector (Clonetech) was used. Experimental variation and transfection efficiency was monitored by cotransfecting pRL-TK (Promega), encoding a renilla luciferase gene, driven by the constitutively active Herpes simplex virus thymidine kinase promoter. Murine T cells were electroporated with the reporter constructs using Mouse T cell kit (AMAXA) and Nucleofector I (AMAXA) according to the manufacturer's protocol. Luciferase activity was quantified with Dual Luciferase Assay Kit (Promega) and a luminometer (Moonlight 3096, BD Biosciences).

### In-silico genomic DNA analysis

The genomic sequences for the twist1 locus of Mus musculus and Homo sapiens were obtained from UCSC Genome Bioinformatics (http://genome.ucsc.edu). To identify putative binding site for DNA binding factors the conserved DNA sequences were submitted to Matinspector at http://www.genomatix.de/matinspector.html.

### Construction of retroviral expression vectors

The twist1 targeting shRNA vector was generated by PCR amplification of a fragment containing the EF1α-promotor and green fluorescent protein (gfp) from pLVTHM, generously provided by D. Trono (Ecole Polytechnique Fédérale de Lausanne, Switzerland). Xbal and EcoRV sites were introduced to replace the corresponding fragment in the retroviral expression plasmid pQCXIX (Clonetech). The sequence targeting twist1 mRNA corresponds to the sequence of Twist-siRNA3 (5'-AAGCTGAGCAAGATTCAGACC-3'). A corresponding scrambled sequence was used as control. The DNA oligonucleotides for shRNA expression were annealed, phosphorylated, and subcloned into the Hpal and Xhol sites of the lentiviral shRNA expression plasmid pLL3.7, generously provided by L. Van Parijs (Massachusetts Institute of Technology, Cambridge, Massachusetts). The fragment of pLL3.7 containing the murine U6-promotor and the shRNA-encoding sequence was amplified by PCR, introducing an additional 5'-Xhol site and ligated into the Sail site of pQXIX-gfp that is located in the inactivated 3'-LTR of the plasmid. For retroviral overexpression the vector GFP-RV(44) was used, kindly provided by K.M. Murphy (Howard Hughes Medical Institute, St. Louis, Missouri). The complete coding sequences of murine T-bet (cDNA generated from Th1-cells), murine twist1 (IMAGE cDNA clone; AccessionID: BC033434-NCBI), and constitutively active IκBαM, lacking phosphorylation sites (pCMV-IκBαM, Clonetech) were amplified by PCR. BglII and Xhol-compatible restriction sites were introduced by the PCR primers for cloning into the unique sites of the vector upstream of the internal ribosome entry site (IRES)-gfp cassette. The accuracy of cloning steps was confirmed by DNA sequencing. Sequences of primers and oligonucleotides for shRNA expression are listed below.

### Immunoblot

Cells were lysed on ice with 1% w/v SDS, 10mM EDTA, 50mM Tris, pH 8,1. Chromatin was sheared by sonification. Equal amounts of protein were resolved by SDS PAGE, then transferred onto polyvinyldifluoride membranes. Membranes were blocked with 5% w/v non-fat dry milk and 0,1% v/v Tween20 in PBS, and probed with a monoclonal Twist-specific antibody (αTwiMab-1)(45) conjugated in house to digoxygenin (Roche Diagnostics), or anti-tubulin-α (DM1A, Calbiochem), followed by incubation with horseradish peroxidase-coupled anti-digoxigenin FAB-fragments (Roche Diagnostics) or anti-mouse (Santa Cruz) secondary antibodies. Individual bands were visualized with enhanced chemiluminescence (Amersham Biosciences) and the Intelligent Dark Box System LAS-3000 (Fujifilm).

### Microarray experiments

Total RNA was extracted using Trizol reagent (Invitrogen). RNA concentration, purity and integrity were assessed with the Agilent 2100 Bioanalyzer, and the RNA 6000 Nano LabChip. Ten micrograms of total RNA was reverse-transcribed, followed by cDNA extraction using a PhaseLock gel (Eppendorf), and precipitation with ethanol and ammonium acetate. Biotinylated cRNA was in vitro transcribed using the MEGAscript high yield transcription kit (Ambion) according to the manufacturer's recommendations. Biotinylated cRNA was fragmented, and the hybridization cocktail was prepared according to Affymetrix protocols (15 µg fragmented biotin-labeled cRNA spiked with Eukaryotic Hybridization control). The Murine Genome U74A version 2, and 430A version 2 GeneChip arrays (Affymetrix) were loaded with the hybridization cocktail, hybridized at 45 oC for 16 h in a rotisserie motor, washed, and stained with streptavidin-phycoerythrin using the Affymetrix GeneChip Fluidics Workstation 400. Arrays were scanned on a Hewlett-Packard Gene Array Scanner (MGU74Av2 arrays) or on an Affymetrix GeneChip Scanner 3000 (MG430Av2 arrays). Data were analyzed using the Microarray Suite 5.0 software (Affymetrix). Microarrays were globally normalized and scaled to a trimmed mean expression value of 200. Quality checks were performed according to the manufacturer's recommendations. All arrays were compared to each other, and a relational database was generated using Microsoft Access software, including the following parameters: expression heights, call for presence of transcripts, p value for presence or absence of transcripts, log2 value of fold change and 95% confidence intervals, call for the significance of differentially expression, and the p value for that call. For each transcript the significance of differential expression between the groups of arrays was calculated using strict Bonferroni corrected Welch t-tests. Significantly differentially expressed genes were filtered according to the following criteria: mean fold change >= 2 or 1,5; difference of means >= 200; p-value <= 0,05; and immunoglobulin genes were excluded.

### Real-time PCR

Total RNA was prepared using NucleoSpin RNA II (Macherey-Nagel) or RNeasy kit (Qiagen). Reverse transcription (TaqMan Reverse Transcription Reagent, Applied Biosystems) was performed in a conventional thermocycler (10 min at 25°C, 40 min at 48°C, and 5 min at 95°C) with 500 ng of total RNA and a 1:1 mixture of oligo(dT) and random hexamer primers. Real-time PCR was performed with the LightCycler instrument using the FastStart DNA Master SYBR Green I kit (Roche Diagnostics). For the normalization of murine and human cDNA the transcripts for the housekeeping genes hypoxanthine guanine phosphoribosyl transferase (HPRT) and ubiquitin ligase H5 (UbcH5) were quantified, respectively. Data were evaluated using Lightcycler software. Relative expression was calculated as follows: Eₜ^{ΔCp target gene (reference - sample)/EhΔCp} housekeeping gene (reference - sample), where Cp represents the crossing point and E represents the reaction efficiency, determined by serial dilution of DNA. Primer sequences are listed in Table S5.

### Figures and Tables

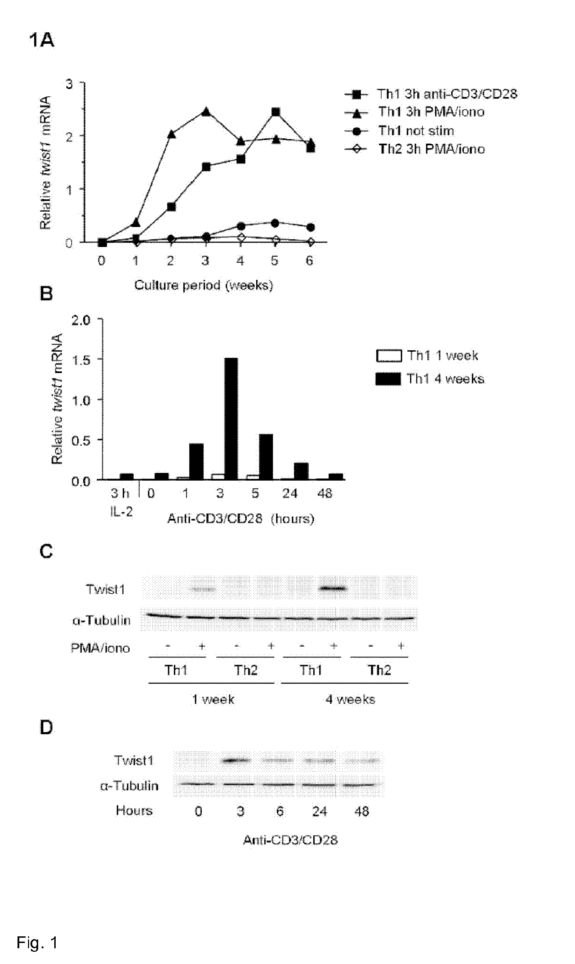

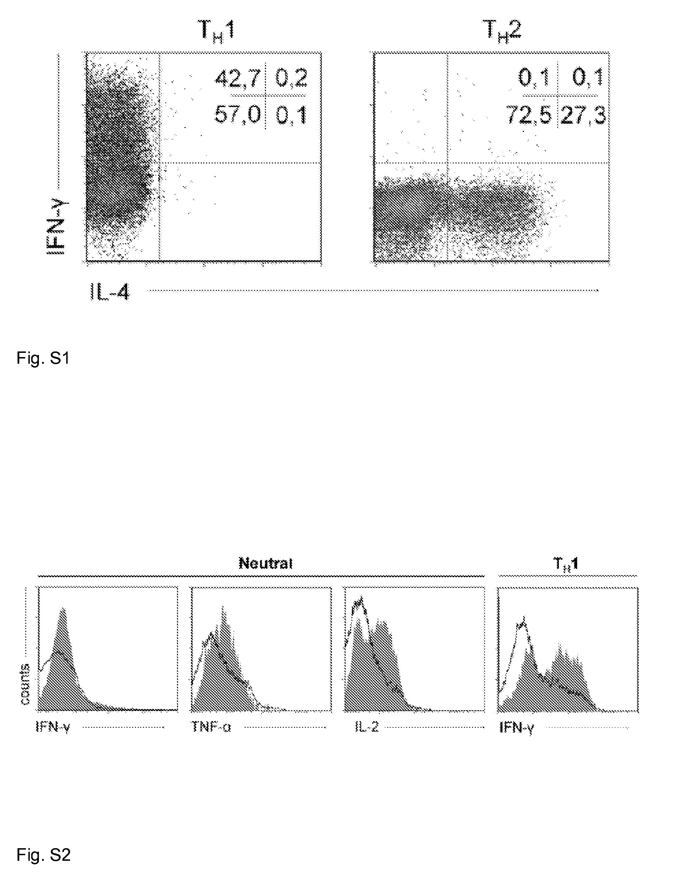

Fig. 1 shows that twist1 expression is induced in Th1 but not Th2 cells. (A) CD4⁺CD62L^{hi} DO11.10 T lymphocytes were repeatedly stimulated in vitro under Th1 or under Th2-polarizing conditions. Functional polarization of Th1 and Th2 cells, i.e. IFN-γ and IL-4 expression, respectively, was confirmed by intracellular immunofluorescence (Fig. S 1). Twist1 mRNA in resting cells 6 d after the last stimulation (Th1; circles) or after 3 h of restimulation with anti-CD3/CD28 and IL-2 (Th1; squares) or PMA/ionomycin and IL-2 (Th1; triangles; Th2; diamonds) was determined by RT PCR and normalized to HPRT. (B) Kinetics of twist1 mRNA expression in 1 (open bars) and 4 (filled bars) w-old Th1 cells, after stimulation with anti-CD3/CD28 and IL-2 or IL-2 alone. (C) Twist1 protein expression in resting (-) and 5 h PMA/ionomycin restimulated (+) 1 and 4-w-old Th1 and Th2 cells. Control: α-Tubulin immunoblot (bottom). (D) Kinetics of Twist1 protein expression in 4-w-old Th1 cells, before and at the indicated time intervals of stimulation with anti-CD3/CD28 and IL-2. Data are representative of two experiments.

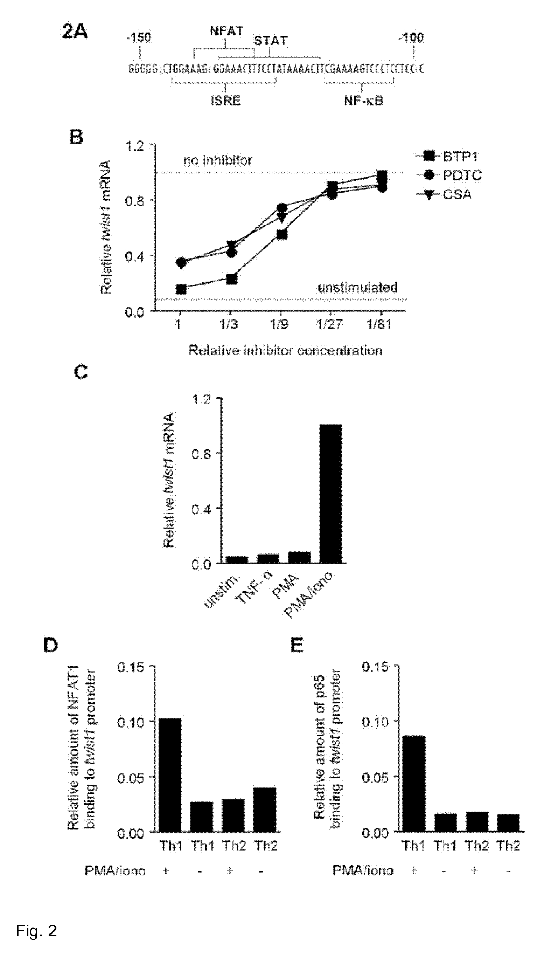

Fig. 2 shows that signaling through NFAT and NF-κB is required for induction of twist1 expression. (A) Comparison of the genomic sequence of the murine and the human proximal twist1 promoter (-150 to -100). The murine sequence is displayed with conserved bases in capital letters. Selected putative DNA-binding motifs are indicated. ISRE (interferon-stimulated response element) (B) Twist1 mRNA in 4-w-old Th1 cells restimulated for 3 h with PMA/ionomycin and IL-2 in the presence of serial dilutions of the NF-κB inhibitor PDTC (circles; starting concentration 50 µM), the NFAT inhibitor BTP1 (squares; starting conc. 100 nM), or the NF-κB and NFAT inhibitor CsA (triangles; starting conc. 30 nM). (C) 4-w-old Th1 cells were restimulated in the presence of IL-2 for 3 h with IL-2 alone (unstimulated), 10 ng/ml TNF-α, PMA, or PMA/ionomycin. The binding of NFAT1 (D) and the NF-κB subunit p65 (E) to the proximal promoter of twist1 was analyzed by chromatin immunoprecipitation (ChIP). 3 to 4-w-old Th1 and Th2 cells either in the resting state (-) or following restimulation with PMA/ionomycin and IL-2 for 1 h (+) were used. The immunoprecipitated DNA was quantified by RT PCR using primers specific for the proximal promoter. The precipitated DNA was normalized to the amount of input DNA. Data are representative of three independent experiments.

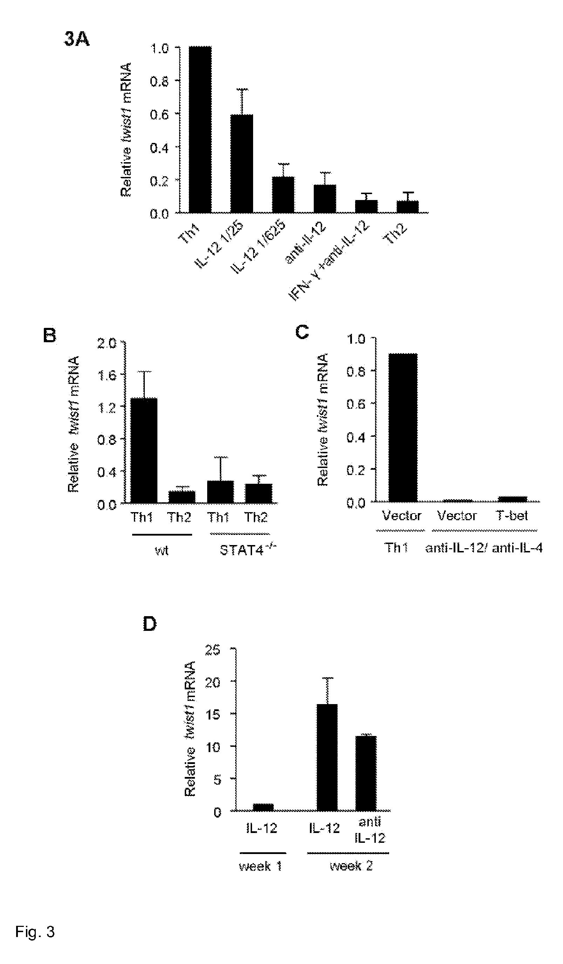

Fig. 3 shows that twist1 induction requires IL-12 signaling via STAT4, but not IFN-γ or T-bet. Twist1 mRNA of Th cells activated for 3 h with PMA/ionomycin and IL-2 was quantified by RT PCR (A-D). (A) CD62L^{hi} DO11.10 Th cells were stimulated for 5 d under Th1-polarizing conditions (5 ng/ml IL-12), reduced IL-12 (1/25; 200pg/ml IL-12; 1/625; 8pg/ml IL-12), in the absence of IL-12 (anti-IL-12, anti-IL4), in the presence of IFN-γ (10 ng/ml IFN-γ, anti-IL-12, anti-IL-4), or under Th2-polarizing conditions The amount of twist1 transcripts induced under Th1-polarizing conditions was set to 1. Data are presented as average ± s.d. of at least three experiments. (B) CD4⁺ cells of STAT4^{-/-} and syngenic BALB/c mice were stimulated with anti-CD3/CD28, and irradiated BALB/c APCs under Th1 (IL-12 and IFN-γ), or under Th2-polarizing conditions for 5 d. Data represent the average ± s.d. of four individual mice each. (C) Naïve D011.10 Th cells were stimulated twice under Th1-polarizing conditions, or in the absence of IL-12. On d 2 the cells were infected with control virus, or a virus encoding t-bet. Infected cells were isolated on d 12 according to expression of the viral marker gene gfp. Data are representative of two experiments. (D) Naïve D011.10 Th cells were stimulated for 5 d with splenic APCs and ova₃₂₇₋₃₃₉ under Th1-polarizing conditions. Cells were restimulated under the same conditions (Th1), or in the presence of anti-IL12. Twist1 transcripts were quantified on d 11. The amount of twist1 mRNA on d 5 was set to 1. Data represent average ± s.d. of three experiments.

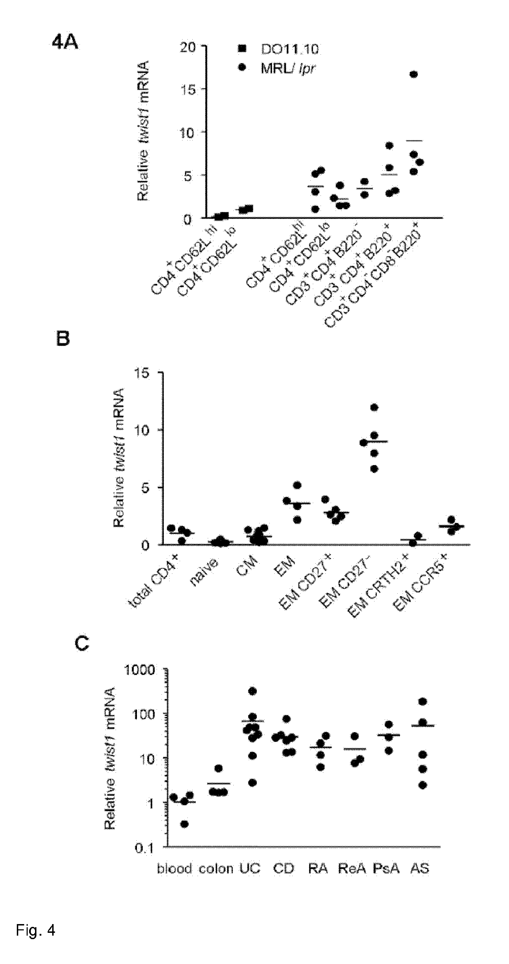

Fig. 4 shows that ex-vivo isolated memory Th cells express twist1. Cells were sorted by flow cytometry and restimulated 3 h with PMA/ionomycin (A-C). (A) Cells were isolated from the spleen and lymph nodes of 8-12-w-old D011.10 mice (squares, each representing a pool of 15 individual mice) and the pooled inguinal and mesenteric lymph nodes of 4-6-month old nephritic MRL/Ipr mice (circles, 1-2 mice each). Of note: 90% of the CD4⁺CD62L^{hi} cells in MRL/Ipr mice represented activated (CD44⁺) cells (data not shown). (B) Twist1 mRNA in peripheral human Th lymphocytes. The mean expression of twist1 mRNA normalized to ubiquitin ligase H5 in total CD3⁺CD4⁺ cells was set to 1. Subpopulations were defined according to expression of the following surface markers: Naïve (CD4⁺CD45RA⁺CCR7⁺), central memory (CM; CD4⁺CD45RA⁻CCR7⁺), effector memory (EM; CD4⁺CD45RA⁻CCR7⁻) with each data point representing one individual healthy donor. (C) Twist1 transcripts in CD3⁺CD4⁺ cells purified from patient material: Blood (total peripheral CD3⁺CD4⁺ cells from healthy donors, see above), colon (non-inflamed colon tissue), UC and CD (endoscopic biopsies from ulcerative colitis and Crohn's disease patients, respectively), RA, ReA, PsA, and AS (synovial fluid from rheumatoid arthritis, reactive arthritis, psoriatic arthritis, and ankylosing spondylitis patients, respectively) with each dot representing one individual patient. Mean twist1 mRNA expression is displayed from patients who were repeatedly sampled.

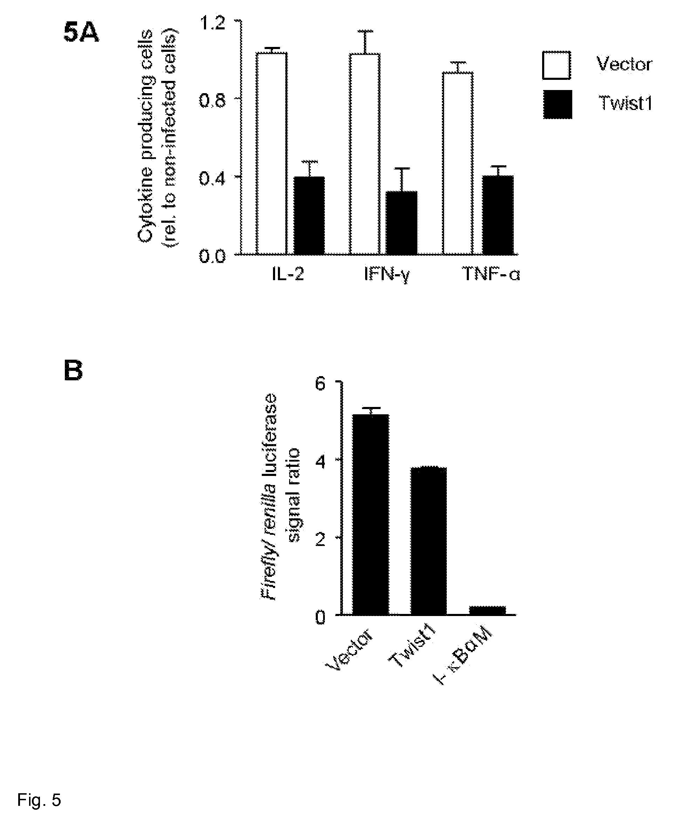

Fig. 5 shows that Twist1 modulates Th1-effector functions. (A) DO11.10 Th cells were stimulated with ova₃₂₇₋₃₃₉ and APC for 5 d. On d 2, cells were infected with control virus (open bars) or twist1-encoding virus (filled bars). On d 6, the resting cells were re-stimulated and stained for intracellular cytokine expression. Frequencies of cytokine expressing cells among infected, i.e. GFP⁺CD4⁺ T cells relative to the non-infected CD4⁺ cells are displayed. Data represent the mean ± s.d. of four independent experiments. (B) DO11.10 Th cells were stimulated with ova₃₂₇₋₃₃₉, APCs, and 1ng/ml IL-12. On d 2 cells were infected with control virus, twist1, or I-κBαM-encoding virus. On d 3, cells were nucleoporated with a mixture of a plasmid encoding renilla luciferase, and a firefly luciferase reporter construct, driven by a NF-κB-responsive promoter (4xκB-luc). Cells were then re-stimulated with PMA/ionomycin for 6 h, sorted according to expression of the viral marker gene gfp, and luciferase signals were quantified in duplicates (mean ± s.d.).

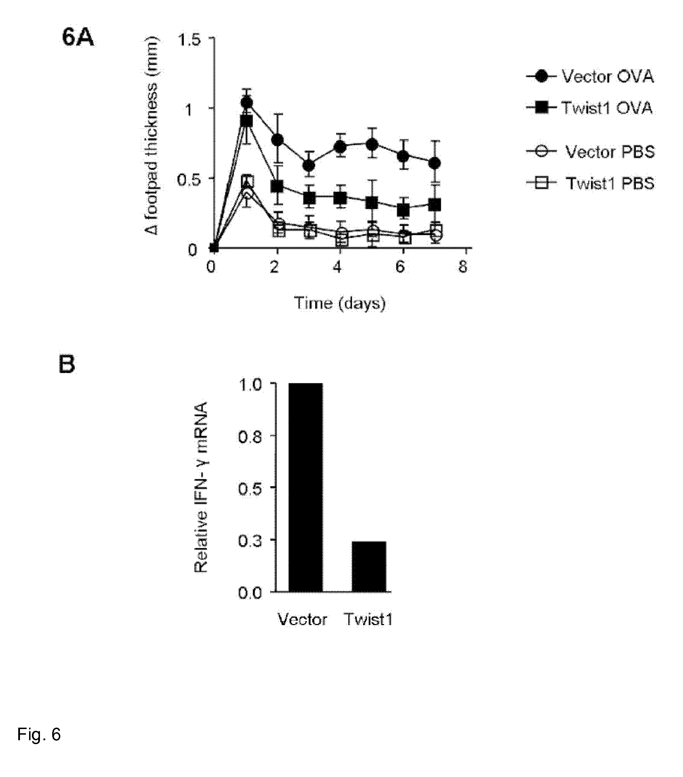

Fig. 6 shows that ectopic twist1 over-expression controls DTH. (A) Naïve DO11.10 Th cells were stimulated with anti-CD3/CD28 under Th1-polarizing conditions. On d 2, cells were infected with control virus (circles) or twist1-encoding virus (squares). On d 6, infected GFP+ were injected i.v. into BALB/c mice. The DTH response was induced by s.c ova₃₂₇₋₃₃₉/IFA injection into the left footpad (filled symbols), and Δ footpad thickness (mean ± s.d.; n = 6) was determined thereafter. Injection of PBS/IFA served as control (open symbols). (B) IFN-γ mRNA expression in transferred GFP⁺ Th1 cells 24 h after DTH induction isolated from the draining popliteal lymph node (left foot).

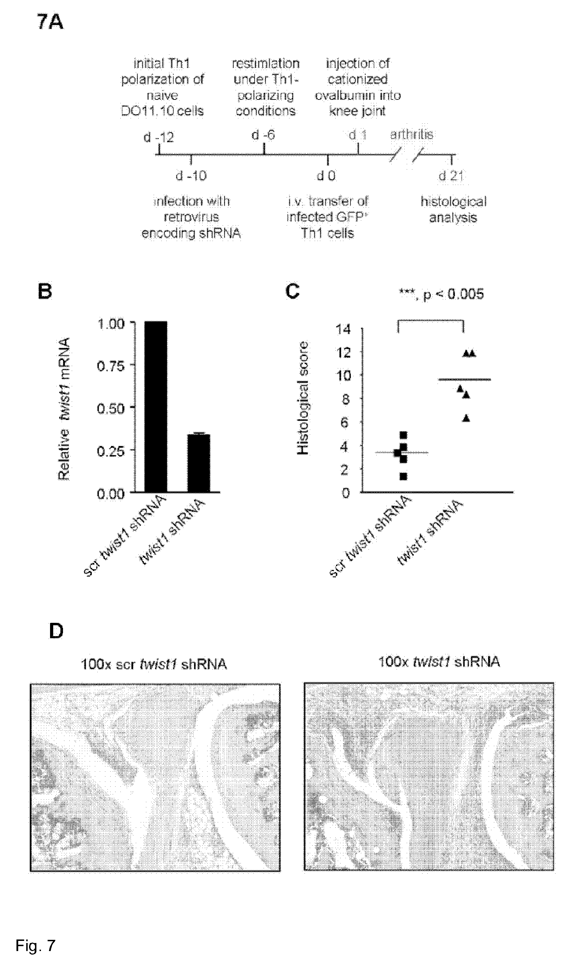

Fig. 7 shows that twist1 knock-down increases inflammatory response in murine arthritis. (A) Experimental scheme. (B) Twist1 mRNA in 3-w-old Th1 expressing twist1-targeting shRNA or control shRNA re-stimulated with PMA/ionomycin. (C) Transfer of Th1 cells expressing a twist1-targeting shRNA leads to a significantly higher histological score in murine arthritis compared to control Th1 cells (d 21) Data are representative of two experiments. (D) Representative hematoxilin/eosin staining of knee joint sections (d21).

Fig. S1 shows representative cytokine profiles of ex vivo-polarized TH1 and TH2 cells. Naïve CD4⁺CD62L^{hi} DO11.10 T_{H} cells cultured for 6 d under T_{H}1 or T_{H}2-polarizing conditions were re-stimulated with PMA/ionomycin and brefeldin A for 5 h, fixed, and stained for intracellular IL-4 and IFN-γ. For cytometric analysis, cells were gated for expression of CD4. Numbers in quadrants indicate percentages of gated cells in each.

Fig. S2 shows that ectopic twist1 overexpression attenuates cytokine expression in Th cells. Representative histograms of cytokine expression in T_{H} cells ectopically expressing twist1 (black line) and control cells (gray filled). The cells displayed were gated for expression of CD4 and the viral marker gene gfp. CD4⁺ DO11.10 lymphocytes were stimulated with ova₃₂₇₋₃₃₉ and APC for 6 d (Neutral) or under TH₁-polarizing conditions (T_{H}1). On d 2 cells were infected with control virus or twist1-encoding virus. On d 6 cells were re-stimulated with PMA/ionomycin in the presence of brefeldin A for 5 h and stained for intracellular cytokine expression.

Table 1 lists genes differentially expressed in pathogenic Th1 cells.

Table 2 lists genes differentially expressed in pathogenic Th2 cells.

Table 3 lists genes differentially expressed upon ectopic twist1 overexpression. Splenic DO11.10 cells were activated in vitro with the cognate peptide ova₃₂₇₋₃₃₉ in the presence of 1ng/ml IL-12 and 1ng/ml IL-2. On d 2 cells were infected with control retrovirus, or twist1-encoding virus. On d 5 cells were sorted according to expression of the viral marker gene gfp. Cells were restimulated with PMA/ionomycin. The transcriptional profiles of duplicates of cultures were compared.

Table S1 lists genes differentially expressed in once versus four times stimulated T_{H}1 cells. Naïve DO11.10 cells were activated under T_{H}1-polarizing conditions. The transcriptional profiles of resting one-week-old T_{H}1 (T_{H}1 1w) cells and resting four-week-old T_{H}1 (T_{H}1 4w) cells were compared using Affymetrix Murine Genome (MG) U74Av2 GeneChip arrays. The Affymetrix probe set ID (Affymetr_No), mean fold change, significance of differential expression (t-test), and mean signal intensity (T_{H}1 1w, T_{H}1 4w) of three arrays per group representing three independent cultures are shown. Genes were filtered according to the following criteria: fold change >= 2; difference of means >= 200; p-value <= 0,05

Table S2 lists clinical characteristics of patients in the study. Erythrocyte sedimentation rate (ESR), C-reactive protein (CRP) Twist1 transcripts in CD3⁺CD4⁺ cells purified from patient material were quantified after 3 h of re-stimulation with PMA/ionomycin. The mean expression of twist1 mRNA in total peripheral CD3⁺CD4⁺ cells from healthy donors was set to 1.

Table S3 lists genes differentially expressed upon ectopic twist1 overexpression. Splenic DO11.10 cells were activated in vitro with the cognate peptide ova₃₂₇₋₃₃₉ in the presence of 1ng/ml IL-12 and 1ng/ml IL-2. On d 2 cells were infected with control virus, or twist1-encoding virus. On d 5 cells were sorted according to expression of the viral marker gene gfp. Cells were re-stimulated for 4 h with PMA/ionomycin. The transcriptional profiles of duplicates of cultures were compared using Affymetrix Murine Genome (MG) 430A 2.0 GeneChip arrays. The Affymetrix probe set ID (Affymetr_No), mean fold change, significance of differential expression (t-test), and mean signal intensity (Vector, Twist1) of two arrays per group are shown. Genes were filtered according to the following criteria: fold change >= 1,5; difference of means >= 200; p-value <= 0,05, and excluding immunoglobulin genes.

Table S4 shows that twist1 knock-down results in a higher inflammatory response in murine arthritis. After i.v. cell transfer of two-week-old GFP⁺ DO11.10 T_{H}1 cells expressing twist1-targeting shRNA (Twist1-5) or control shRNA (scrT1-5), arthritis was induced in the recipient SCID mice by intraarticular injection of cationized ovalbumin into the knee joint. 21 days later, knee joint sections were stained for hematoxilin/ eosin and scored for exudates, granulocyte infiltration (gran. inf. SM), hyperplasia (hyperpl.), fibroblast proliferation/mononuclear cell infiltration (mono. inf. SM), periarticular mononuclear cell infiltration (peri. mino.) (each scoring 0-3), bone/cartilage destruction (scoring 0-4) and additional score of 1 for fibrin deposition and periarticular granulocyte infiltration (periart. gran), resulting in a maximum score of 21.

Table S5 lists primer sequences for real-time PCR.

### Example 1: Twist1 is transiently expressed in repeatedly activated Th1 cells

To define transcriptional changes during Th1 memory cell differentiation we compared the global gene expression of murine Th1 cells, activated once or on a weekly basis repeatedly with antigen. Naïve, CD4⁺CD62L^{hi} T lymphocytes, expressing the transgenic D011.10 TCR specific for ovalbumin were activated in vitro with splenic APCs and the cognate peptide ova₃₂₇₋₃₃₉ under conditions that induce functional differentiation into Th1 cells, i.e. addition of IL-12 and blocking antibodies specific for IL-4. The transcriptional profiles of once and four times stimulated Th1 cells were compared using high-density DNA microarrays. Among the 17 genes differentially expressed by a factor of two or more was twist1. Expression of twist1 was up-regulated 38-fold in four times versus once stimulated Th1 cells (Table S1).

Twist1 expression in Th1, but not Th2 cells was confirmed by real-time PCR and immunoblot analysis (Fig. 1). Twist1 mRNA expression in resting Th1 cells correlated with their age in vitro and the number of re-stimulations they had experienced. Expression was further enhanced three hours after polyclonal stimulation either with PMA and the Ca²⁺ ionophore ionomycin, or with CD3 and CD28-specific antibodies (Fig. 1 A). When determined three hours after re-stimulation with anti-CD3/CD28 antibodies, expression of twist1 was close to the detection limit in naïve Th cells. Expression increased about 15-fold during the first Th1-polarizing stimulation, another 10-fold during the second stimulation, then another 3-fold during the third stimulation, reaching the maximum level after the fourth stimulation, and remained stable thereafter. With PMA/ionomycin re-stimulation, maximum levels of twist1 mRNA were reached already after two rounds of stimulation. In Th1 cells, upon anti-CD3/CD28 re-stimulation, twist1 mRNA expression was up-regulated within the first hour, reaching maximum levels after three hours, and decreasing again thereafter (Fig. 1 B). Expression of twist1 mRNA was also detectable in re-stimulated Th2 cells, but its level remained 30-fold lower than in Th1 cells. Twist1 protein was detectable in 6-day old, re-stimulated Th1 cells. In 4-week old Th1 cells, expression was enhanced. In resting Th1 cells and in reactivated or resting Th2 cells, Twist1 was not detectable by immunoblotting (Fig. 1 C). In accordance with expression of twist1 mRNA, Twist1 protein expression peaked already three hours after reactivation, then ceased, with detectable levels still expressed 48 hours after re-stimulation (Fig. 1 D) but no longer six days after re-stimulation. Twist2 was not expressed in the Th1 and Th2 cells analyzed here, as determined by real-time PCR.

### Example 2: Control of twist1 expression in Th1 lymphocytes

Phylogenetic comparison of the proximal promoter of twist1 from man and mouse (Fig. 2 A) revealed conserved sequence motifs which qualify as binding sites for the transcription factors NFAT, STAT, and NF-κB. While NFAT and NF-κB could transmit TCR signals to the promoter, the STAT-binding site could be a target of Th1-polarizing signals, i.e. IL-12 and/or IFN-γ. With respect to control of twist1 expression by TCR signaling in Th1 cells, NF-κB and NFAT were identified as the relevant transcription factors by specific inhibition and chromatin immunoprecipitation (ChIP). The NF-κB inhibitor pyrrolidine dithiocarbamate (PDTC), the NFAT-specific inhibitory 3,5-bistrifluoromethyl pyrazole derivative BTP1, and the calcineurin-inhibitor cyclosporine A (CsA), blocking both NFAT and NF-κB, all blocked the PMA/ionomycin mediated up-regulation of twist1 mRNA expression in a dose-dependent fashion (Fig. 2 B). This shows that both NFAT and NF-κB are required for induction of twist1 expression in Th1 cells, a result that was confirmed by observing that stimulation with either TNF-α or PMA alone, both activating NF-κB but not NFAT, did not induce twist1 expression, which in addition required the Ca²⁺ ionophore ionomycin, activating NFAT via calcineurin (Fig. 2 C). The requirement of NFAT for induction of twist1 expression in Th1 cells distinguishes control of twist1 expression in Th cells from its control in fibroblasts, where TNF-α-induced activation of NF-κB is sufficient to induce expression.

By ChIP, the specific binding of NFAT1 and the transactivating NF-κB subunit p65 to the twist1 promoter of repeatedly stimulated Th1 cells was evident one hour after reactivation (Fig. 2 D-E). In Th2 cells, no activation-induced binding of NFAT1 or NF-κB to the twist1 promoter was detectable, emphasizing that NFAT1 and NF-κB are required, but not sufficient to induce transcription of twist1 in Th cells.

The specific expression of twist1 by Th1 cells raised the question which of the Th1-polarizing signals is the specific inductive signal in these cells, cooperating with NFAT1 and NF-κB. IL-12, the primordial signal for Th1 differentiation induced twist1 expression in a dose-dependent fashion (Fig. 3 A). This effect is direct, and not indirect through induction of IFN-γ and STAT1 signaling, since addition of IFN-γ in the absence of IL-12 did not induce twist1 expression. IL-12 induces twist1 expression via STAT4. When CD4⁺ T lymphocytes of STAT4-deficient mice were stimulated in the presence of IL-12 and IFN-γ, no induction of twist1 expression was detectable (Fig. 3 B). Neither IFN-γ and its signal transducer STAT1, nor T-bet, a T box transcription factor sufficient to induce Th1 differentiation are involved in expression control of twist1. Ectopically expressed T-bet did not induce twist1 expression in the absence of IL-12 (Fig. 3 C). IL-12/STAT4 is primarily required for the initial induction of twist1 expression upon Th1 polarization to imprint the gene for re-expression. Th1 cells polarized for one week with IL-12, and reactivated for another week in the absence of IL-12, showed a 12-fold increased TCR activation-dependent expression of twist1 over one week stimulated Th1 cells, as compared to a 16-fold increase in the presence of IL-12 (Fig. 3 D).

### Example 3: Th cell-specific twist1 expression in-vivo

Expression of twist1 mRNA was low in PMA/ionomycin re-stimulated CD4+CD62Lhi naïve and CD4⁺CD62L^{lo} memory cells isolated from the spleen and lymph nodes of healthy DO11.10 mice, kept under specific pathogen-free conditions (Fig. 4 A). Weak twist1 expression was also detected in PMA/ionomycin reactivated CD4⁺ T cells from peripheral blood of healthy donors (Fig. 4 B). As compared to the reference expression of twist1 in total peripheral human Th lymphocytes, twist1 transcripts were enhanced 3-fold in effector memory (EM, i.e. CD45RA⁻CCR7⁻) Th cells, and 8-fold in 'terminally' differentiated CD27⁻ EM Th cells. Naïve (CD45RA⁺CCR7⁺) and central memory (CM, i.e. CD45RA⁻CCR7⁺) Th lymphocytes had a lower expression level compared to unseparated Th cells. In contrast to CCR5⁺ Th1 EM, CRTh2⁺ Th2 EM cells showed no significant expression of twist1. Thus, the phenotype of human peripheral twist1 expressing Th cells is that of repeatedly re-stimulated Th1 EM cells.

Since twist1 expression increases in Th1 EM cells with the number of re-stimulations, we analyzed twist1 expression of Th cells in a murine model of chronic inflammation with a proposed involvement of Th1 cells. In CD4⁺CD62L^{lo} memory Th cells of lymph nodes of 4-6 month old nephritic lupus-prone MRL/Ipr mice twist1 mRNA was up-regulated about 3-fold, as compared to CD4⁺CD62L^{lo} Th cells of D011.10 spleen (Fig. 4 A). Twist1 expression was even further enhanced in CD3⁺B220⁺ T cells from MRL/Ipr mice, which presumably represent chronically activated Th lymphocytes.

Th cells isolated from inflamed tissue of patients with various chronic inflammatory diseases showed high PMA/ionomycin-inducible twist1 expression. CD3⁺CD4⁺ Th cells were isolated from inflamed tissues of patients suffering from inflammatory bowel disease or rheumatic diseases, re-stimulated, and analyzed for twist1 expression (Fig. 4 C, Table S2). Th cells isolated from non-inflamed surgical colon specimens of control patients did not show enhanced twist1 expression, as compared to expression in peripheral Th cells. Although highly variable, twist1 transcripts were increased up to 400-fold in Th cells isolated from the synovial fluid of inflamed joints of patients with rheumatoid arthritis or spondyloarthropathies, and in Th cells isolated from mucosal endoscopic biopsies and surgical specimens of patients suffering from Crohn's disease or ulcerative colitis. Twist1 mRNA expression in patients with persistent inflammation of colon or synovia, who were repeatedly sampled, remained in the same range over up to 18 months (Table S2). Among T cells isolated from the inflamed tissue, only CD4+ Th cells showed enhanced twist1 expression. CD3⁺CD4⁻cells, i.e. cytotoxic (Tc) lymphocytes, expressed twist1 transcript levels lower than the reference value of total peripheral Th cells.

### Example 4: Functional modulation of Th1 cells by twist1

The impact of twist1 on the function of Th1 EM cells was analyzed by ectopic overexpression of twist1 in murine DO11.10 Th cells. A global view on twist1-induced modulation of gene expression in Th1 cells is provided in Table 3. Of the 14.000 genes analyzed for transcription, 58 were differentially expressed by a factor of 1.5 or more, between activated Th1 cells expressing twist1 ectopically or not. These genes fall into 4 groups, with respect to their presumptive function. The first group comprises 17 genes which are involved in cell activation and apoptosis, 11 genes are involved in cell adhesion and motility, 13 genes relate to the chemokine/cytokine repertoire of Th1 cells, and 17 genes are of metabolic or undefined relevance.

With respect to activation and proliferation, ectopic twist1 primarily induced genes that have been reported to inhibit T cell receptor signaling (programmed cell death 4 (pdcd4) and tescalin) and the anti-proliferative cellular repressor of E1A-stimulated genes (creg1) whose product is secreted. Twist1 inhibited expression of genes promoting cell activation and cytokine expression in T cells: growth factor independent 1(gfi1), SLAM family member 6 (slamf6, also known as Ly-108), membrane-spanning 4-domains A, 4B (ms4a4b), pro-inflammatory cyclic AMP (cAMP)-specific phosphodiesterase 4b (pde4b), the regulator of G-protein signaling 2 (rgs2), and lymphocyte-activation gene 3 (lag3), a gene blocking memory cell formation. Twist1 negatively regulated transformation related protein 53 inducible nuclear protein 1 (trp53inp1, also known as stress-induced protein) a gene reported to enhance p53-induced apoptosis, and caspase 6, involved in execution of apoptosis.

Twist1 modulation of expression of genes involved in motility and adhesion of Th1 cells presumably results in immobilizing the Th1 cells in inflamed tissue. Ectopically expressed twist1 reduced transcript levels of matrix metalloproteinase 13 (also known as collagenase-1), a disintegrin and metallopeptidase domain 8 (adam8), and the collagen-binding receptor discoidin domain 1 (ddr1), genes favoring tissue destruction.

Ectopic twist1 expression also regulated genes determining effector functions of Th1 cells. Twist1 reduced expression of the chemokine (C-X-C motif) receptor 6 (cxcr6), and of the Th1-related pro-inflammatory chemokine genes ccl3, ccl4, and xcl1 (also known as MIP-1α, MIP-1β, and lymphotactin, respectively), but enhanced expression of ccl5 (RANTES). Twist1 impaired cytokine signaling through enhanced expression of the decoy IL-1 receptor, type II, the suppressor of cytokine signaling (socs) 1 and 2, and repression of the IL-12 signal transmitter janus kinase 2 (jak2), the latter qualifying twist1 as part of a negative feedback loop with respect to IL-12 signaling. Twist1 also attenuated expression of the Th1 effector cytokine genes il-2, ifn-γ and tnf-α by a factor of up to 1.6 (Table 3). This moderate reduction of mRNA levels had a drastic effect on protein expression. Ectopic twist1 expression reduced the frequencies of cells expressing TNF-α, IFN-γ, or IL-2 to 40-50% of the controls (Fig. 5 A, and Figure S 2). The cytokines IL-4 and IL-10 were only marginally expressed by the Th cells analyzed, whether they expressed twist1 or not. Twist1 in Th1 cells thus acts as an endogenous regulator limiting the pro-inflammatory potential of Th1 cells in face of continuous presence of antigen, i.e. repeated activation of NFAT and NF-κB.

The molecular basis of the regulation of NF-κB-dependent cytokine genes by twist1 in Th1 cells could be binding of Twist1 to regulatory elements of target genes, or direct inhibition of NF-κB. In macrophages, inhibition of TNF-α expression has been shown to require the integrity of E-boxes, the DNA-binding motif of Twist proteins, which are located within the TNF-α promoter, while in COS cells, ectopically expressed Twist proteins directly interacted with the p65 subunit of NF-κB, and inhibited its function. In activated primary murine Th cells a constitutively active inhibitor of NF-κB (I-κBaM) but not ectopically expressed twist1 or was able to repress activation-induced transcription of an NF-κB-reporter construct lacking E-boxes (Fig. 5 B), while the expression of endogenous cytokine genes was attenuated to the same degree. This result strongly suggests that twist1 directly regulates gene expression of Th1 cells by binding to E-boxes. Functional NF-κB signaling for the generation and survival of Th memory cells is not inhibited by twist1.

### Example 5: Twist1 regulates Th1 mediated inflammation

In a murine transfer model of ovalbumin-specific delayed-type hypersensitivity the effect of twist1 expression on the inflammation induced by transferred Th1 cells was analyzed. Ovalbumin-specific Th1 cells, ectopically overexpressing twist1 or not, were transferred intravenously into naïve BALB/c mice. After 1 day, ovalbumin/IFA was injected into one footpad, and swelling of this footpad was measured thereafter. Th1 cells overexpressing twist1 showed a significantly reduced induction of footpad swelling, starting from day 2, when compared to control Th1 cells (Fig. 6 A). Transferred Th cells overexpressing twist1, when re-isolated from the draining lymph nodes of the host, expressed 4-fold reduced levels of IFN-γ mRNA as compared to the control cells (Fig. 6 B).

Autoregulation of Th1-mediated inflammation by twist1 was also analyzed in a murine model of antigen-induced arthritis (Fig. 7 A), in which endogenous expression of twist1 in Th cells was silenced by RNA interference. Murine D011.10 Th1 cells were infected with a retrovirus encoding a small hairpin RNA (shRNA) targeting twist1 or a corresponding scrambled control shRNA. Twist1-specific shRNA reduced the level of activation-induced endogenous twist1 transcripts in Th1 cells to approximately 30% of the control value (Fig. 7 B). Two-week-old Th1 cells expressing shRNAs were intravenously injected into SCID mice. One day after cell transfer, arthritis was induced by injection of cationized ovalbumin into the knee joint and histological analysis was performed 3 weeks after induction of arthritis, i.e. in the chronic phase of inflammation. Twist1 knockdown in Th1 cells resulted in a significantly higher histological score of inflammation and tissue destruction (Fig. 7 C). In particular, infiltration of granulocytes and monocytes into the inflamed tissue of the knee joint was drastically enhanced (Fig. 7 D and Table S4).

**Table 1**

| **Gene name** | **Accession No.** |
|---|---|
| hydroxyacyl-Coenzyme A dehydrogenase type II | NM_016763 |
| 2'-5' oligoadenylate synthetase 3 | AB067534 |
| a disintegrin and metalloprotease domain 8 | NM_007403 |
| activating transcription factor 3 | BC019946 |
| adipose differentiation related protein | NM_007408 |
| annexin A1 | NM_010730 |
| B-cell leukemia/lymphoma 2 related protein A1a | L16462 |
| beta-site APP-cleaving enzyme 2 | BB348062 |
| bone marrow stromal cell antigen 1 | AI647987 |
| cathepsin D | NM_009983 |
| chemokine (C-C motif) ligand 1 | NM_011329 |
| chemokine (C-C motif) ligand 5 | NM_013653 |
| chemokine (C-C motif) receptor 1 | AV231648 |
| chemokine (C-X-C motif) receptor 6 | NM_030712 |
| clusterin | NM_013492 |
| coagulation factor II (thrombin) receptor | AV024285 |
| colony stimulating factor 1 (macrophage) | M21149 |
| colony stimulating factor 2 (granulocyte-macrophage) | X03019 |
| cyclin D3 | NM_007632 |
| cyclin-dependent kinase inhibitor 1A (P21) | NM_007669 |
| cysteinyl leukotriene receptor 1 | BC027102 |
| cytoplasmic FMR1 interacting protein 1 | NM_011370 |
| diphtheria toxin receptor | L07264 |
| ectonucleoside triphosphate diphosphohydrolase 1 | B1151440 |
| endothelial cell-specific molecule 1 | BC020038 |
| epithelial V-like antigen | BC015076 |
| fibrinogen-like protein 2 | BF136544 |
| FK506 binding protein 5 | U16959 |
| RIKEN full-length enriched, 7 days neonate cerebellum | |
| Mus musculus cDNA clone A730098C13 3- similar to | |
| L16904 Mouse zinc-finger protein (Mfg-2) mRNA, mRNA | |
| sequence | BB261287 |
| glypican 1 | NM_016696 |
| granulin | M86736 |
| granzyme A | NM_010370 |
| granzyme C | NM_010371 |
| granzyme E | NM_010372 |
| granzyme F | NM_010374 |
| granzyme G | NM_010375 |
| granzyme K | AB032200 |
| hepatitis A virus cellular receptor 2, Tim3 | AF450241 |
| histone 1, H4h | NM_013550 |
| homeobox only domain | AK009007 |
| huntingtin interacting protein 1 | BB794880 |
| interferon gamma | K00083 |
| interferon induced transmembrane protein 1 | BC027285 |
| interferon, alpha-inducible protein | AK019325 |
| interferon-induced protein with tetratricopeptide repeats 1 | NM_008331 |
| interleukin 1 receptor, type II | NM_010555 |
| interleukin 1 receptor-like 1 | D13695 |
| interleukin 10 | NM_010548 |
| interleukin 18 receptor accessory protein | NM_010553 |
| killer cell lectin-like receptor subfamily C, member 1 | AF106008 |
| killer cell lectin-like receptor subfamily G, member 1 | NM_016970 |
| killer cell lectin-like receptor, subfamily A, member 3 | U49865 |
| killer cell lectin-like receptor, subfamily D, member 1 | NM_010654 |
| lectin, galactoside-binding, soluble, 3 binding protein | NM_011150 |
| lymphocyte antigen 6 complex, locus I | AF232024 |
| lymphotoxin A | NM_010735 |
| MAD homolog 3 (Drosophila) | B1150236 |
| mannose-6-phosphate receptor, cation dependent | NM_010749 |
| matrix metalloproteinase 9 | NM_013599 |
| membrane-spanning 4-domains, subfamily A, member 4B | NM_029499 |
| membrane-spanning 4-domains, subfamily A, member 4C | NM_022429 |
| musculin | NM_010827 |
| natural killer cell group 7 sequence | NM_024253 |
| neoplastic progression 3 | BC011325 |
| nuclear protein 1 | NM_019738 |
| olfactory receptor 672 | NM_020292 |
| osteoclast stimulating factor 1 | U58888 |
| oxidized low density lipoprotein (lectin-like) receptor 1 | NM_138648 |
| perforin 1 (pore forming protein) | M23182 |
| phospholipase C, gamma 2 | AW546508 |
| pleckstrin | AF181829 |
| pleckstrin homology-like domain, family A, member 1 | NM_009344 |
| potassium channel tetramerisation domain containing 12 | BM220945 |
| potassium channel, subfamily K, member 5 | AF319542 |
| potassium inwardly-rectifying channel, subfamily J, | |
| member 8 | NM_008428 |
| preproenkephalin 1 | M13227 |
| programmed cell death 1 | NM_008798 |
| programmed cell death 1 ligand 2 | NM_021396 |
| protein kinase inhibitor beta, cAMP dependent, testis | |
| specific | AV047342 |
| protein tyrosine phosphatase, receptor type, E | U35368 |
| RAR-related orphan receptor alpha | B1660199 |
| retinoic acid induced 14 | NM_030690 |
| runt related transcription factor 2 | D14636 |
| S100 calcium binding protein A4 | D00208 |
| secreted phosphoprotein 1 | NM_009263 |
| serine (or cysteine) proteinase inhibitor, clade B, member | |
| 6a | NM_009254 |
| serine (or cysteine) proteinase inhibitor, clade E, member | |
| 2 | NM_009255 |
| serine/threonine kinase 32C | BB320288 |
| serum amyloid A 3 | NM_011315 |
| solute carrier family 17 (sodium-dependent inorganic | |
| phosphate cotransporter), member 6 | NM_080853 |
| solute carrier family 37 (glycerol-3-phosphate transporter), | |
| member 2 | BC022752 |
| solute carrier family 38, member 4 | NM_027052 |
| suppressor of cytokine signaling 3 | NM_007707 |
| syndecan binding protein (syntenin) 2 | BC005556 |
| transmembrane protein 4 | NM_019953 |
| tumor differentially expressed 1 | BM239368 |
| tumor necrosis factor | NM_013693 |
| tumor necrosis factor (ligand) superfamily, member 11 | AB032771 |
| tumor necrosis factor (ligand) superfamily, member 6 | NM_010177 |
| tumor necrosis factor (ligand) superfamily, member 7 | NM_011617 |
| twist gene homolog 1 (Drosophila) | NM_011658 |

**Table 2**

| **Gene name** | **Accession No.** |
|---|---|
| sprouty homolog 1 (Drosophila) | NM_011896 |
| tumor necrosis factor (ligand) superfamily, member 11 | AB032771 |
| v-raf-1 leukemia viral oncogene 1 | AB057663 |
| 2'-5' oligoadenylate synthetase 3 | AB067534 |
| stanniocalcin 2 | AF031035 |
| hepatocyte growth factor | AF042856 |
| leukemia inhibitory factor | AF065917 |
| aldo-keto reductase family 1, member C12 | AF177041 |
| G protein-coupled receptor 105 | AF177211 |
| pleckstrin | AF181829 |
| calcitonin receptor-like | AF209905 |
| tripartite motif protein 30 | AF220015 |
| lymphocyte antigen 6 complex, locus I | AF232024 |
| potassium channel, subfamily K, member 5 | AF319542 |
| interleukin 24 | AF333251 |
| activating transcription factor 5 | AF375476 |
| hepatitis A virus cellular receptor 2 | AF450241 |
| growth arrest and DNA-damage-inducible 45 beta | AI323528 |
| amyloid beta (A4) precursor-like protein 1 | AI848048 |
| vitamin K epoxide reductase complex, subunit 1 | AK003237 |
| ring finger protein 128 | AK004847 |
| transmembrane 7 superfamily member 3 | AK010720 |
| solute carrier family 37 (glycerol-3-phosphate transporter), member 3 | AK012071 |
| inhibitor of DNA binding 2 | AK013239 |
| interferon, alpha-inducible protein | AK019325 |
| coagulation factor II (thrombin) receptor | AV024285 |
| hydroxyprostaglandin dehydrogenase 15 (NAD) | AV026552 |
| FBJ osteosarcoma oncogene | AV026617 |
| protein kinase inhibitor beta, cAMP dependent, testis specific | AV047342 |
| granulin | AV166504 |
| chemokine (C-C motif) receptor 1 | AV231648 |
| alanine and arginine rich domain containing protein | AV256613 |
| G protein-coupled receptor 43 | AV370830 |
| selenoprotein M | AY043488 |
| H2A histone family, member Z | AY074806 |
| RIKEN cDNA 2310061 N23 gene | AY090098 |
| triple functional domain (PTPRF interacting) | BB080177 |
| regulator of G-protein signaling 16 | BB100249 |
| UDP-Gal:betaGlcNAc beta 1,3-galactosyltransferase, polypeptide 2 | BB223909 |
| ELOVL family member 5, elongation of long chain fatty acids (yeast) | BB254141 |
| serine/threonine kinase 32C | BB320288 |
| solute carrier family 35 (UDP-glucuronic acid/UDP-N-acetylgalactosamine dual | |
| transporter), member D1 | BB409668 |
| RIKEN cDNA C030046M14 gene | BB622792 |
| TCDD-inducible poly(ADP-ribose) polymerase | BB707122 |
| ASF1 anti-silencing function 1 homolog B (S. cerevisiae) | BC003428 |
| Lutheran blood group (Auberger b antigen included) | BC004826 |
| twisted gastrulation homolog 1 (Drosophila) | BC004850 |
| S100 calcium binding protein A1 | BC005590 |
| 2'-5' oligoadenylate synthetase 1 G | BC018470 |
| endothelial cell-specific molecule 1 | BC020038 |
| interferon-stimulated protein | BC022751 |
| solute carrier family 37 (glycerol-3-phosphate transporter), member 2 | BC022752 |
| schlafen 8 | BC024709 |
| cyclin-dependent kinase inhibitor 2C (p18, inhibits CDK4) | BC027026 |
| microtubule-associated protein, RP/EB family, member 2 | BC027056 |
| cysteinyl leukotriene receptor 1 | BC027102 |
| sulfite oxidase | BC027197 |
| insulin-like growth factor binding protein 7 | BE446893 |
| fibrinogen-like protein 2 | BF136544 |
| sterol O-acyltransferase 1 | BG064396 |
| Kruppel-like factor 4 (gut) | BG069413 |
| retinol dehydrogenase 10 (all-trans) | BG073496 |
| Recombinant antineuraminidase single chain Ig VH and VL domains (LOC56304), | |
| mRNA | BG966217 |
| ectonucleoside triphosphate diphosphohydrolase 1 | B1151440 |
| neoplastic progression 3 | BM210600 |
| potassium channel tetramerisation domain containing 12 | BM220945 |
| colony stimulating factor 1 (macrophage) | BM233698 |
| protein tyrosine phosphatase, non-receptor type 13 | BM236743 |
| S100 calcium binding protein A4 | D00208 |
| runt related transcription factor 2 | D14636 |
| a disintegrin-like and metalloprotease (reprolysin type) with thrombospondin type 1 | |
| motif, 1 | D67076 |
| heat shock protein 1 B | M12573 |
| preproenkephalin 1 | M13227 |
| Wilms tumor homolog | M55512 |
| gastrin releasing peptide receptor | M57922 |
| a disintegrin and metalloprotease domain 8 | NM_007403 |
| suppressor of cytokine signaling 2 | NM_007706 |
| chemokine (C-C motif) receptor 8 | NM_007720 |
| colony stimulating factor 2 receptor, beta 2, low-affinity (granulocyte-macrophage) | NM_007781 |
| cathepsin H | NM_007801 |
| early growth response 1 | NM_007913 |
| GLI-Kruppel family member GLI3 | NM_008130 |
| interferon-induced protein with tetratricopeptide repeats 1 | NM_008331 |
| lymphocyte-activation gene 3 | NM_008479 |
| melatonin receptor 1A | NM_008639 |
| phosphatidylinositol-4-phosphate 5-kinase, type 1 alpha | NM_008846 |
| serine (or cysteine) proteinase inhibitor, clade E, member 1 | NM_008871 |
| S100 calcium binding protein A13 | NM_009113 |
| chemokine (C-X-C motif) ligand 2 | NM_009140 |
| serine (or cysteine) proteinase inhibitor, clade E, member 2 | NM_009255 |
| secreted phosphoprotein 1 | NM_009263 |
| pleckstrin homology-like domain, family A, member 1 | NM_009344 |
| T-cell lymphoma invasion and metastasis 1 | NM_009384 |
| amphiregulin | NM_009704 |
| carbonic anhydrase 2 | NM_009801 |
| cyclin D2 | NM_009829 |
| cathepsin D | NM_009983 |
| tumor necrosis factor (ligand) superfamily, member 6 | NM_010177 |
| ganglioside-induced differentiation-associated-protein 10 | NM_010268 |
| H2A histone family, member X | NM_010436 |
| hydroxysteroid (17-beta) dehydrogenase 7 | NM_010476 |
| interleukin 5 | NM_010558 |
| keratin complex 1, acidic, gene 18 | NM_010664 |
| annexin A1 | NM_010730 |
| lymphocyte antigen 6 complex, locus C | NM_010741 |
| interleukin 1 receptor-like 1 | NM_010743 |
| musculin | NM_010827 |
| serine (or cysteine) proteinase inhibitor, clade B, member 2 | NM_011111 |
| phospholipid transfer protein | NM_011125 |
| POU domain, class 2, associating factor 1 | NM_011136 |
| peroxisome proliferator activated receptor gamma | NM_011146 |
| lectin, galactoside-binding, soluble, 3 binding protein | NM_011150 |
| serum amyloid A 3 | NM_011315 |
| chemokine (C-C motif) ligand 1 | NM_011329 |
| serine (or cysteine) proteinase inhibitor, clade F, member 1 | NM_011340 |
| tumor necrosis factor (ligand) superfamily, member 7 | NM_011617 |
| ubiquitin specific protease 18 | NM_011909 |
| tumor necrosis factor | NM_013693 |
| histone 1, H1c | NM_015786 |
| hydroxyacyl-Coenzyme A dehydrogenase type II | NM_016763 |
| interferon regulatory factor 7 | NM_016850 |
| atonal homolog 7 (Drosophila) | NM_016864 |
| solute carrier family 7 (cationic amino acid transporter, y+ system), member 8 | NM_016972 |
| tubulin, alpha 8 | NM_017379 |
| activity regulated cytoskeletal-associated protein | NM_018790 |
| cytochrome P450, family 11, subfamily a, polypeptide 1 | NM_019779 |
| transmembrane protein 4 | NM_019953 |
| resistin like alpha | NM_020509 |
| parathyroid hormone | NM_020623 |
| interleukin 4 | NM_021283 |
| programmed cell death 1 ligand 1 | NM_021893 |
| pericentriolar material 1 | NM_023662 |
| natural killer cell group 7 sequence | NM_024253 |
| ORM1-like 3 (S. cerevisiae) | NM_025661 |
| solute carrier family 39 (metal ion transporter), member 8 | NM_026228 |
| solute carrier family 38, member 4 | NM_027052 |
| chemokine (C-X-C motif) receptor 6 | NM_030712 |
| Jun dimerization protein 2 | NM_030887 |
| interleukin 6 | NM_031168 |
| leucine zipper transcription factor-like 1 | NM_033322 |
| tubby like protein 4 | NM_054040 |
| solute carrier family 17 (sodium-dependent inorganic phosphate cotransporter), member 6 | NM_080853 |
| dual specificity phosphatase 16 | NM_130447 |
| immediate early response 3 | NM_133662 |
| oxidized low density lipoprotein (lectin-like) receptor 1 | NM_138648 |
| SET and MYND domain containing 1 | U76372 |
| histone 1, H2ae | W91024 |

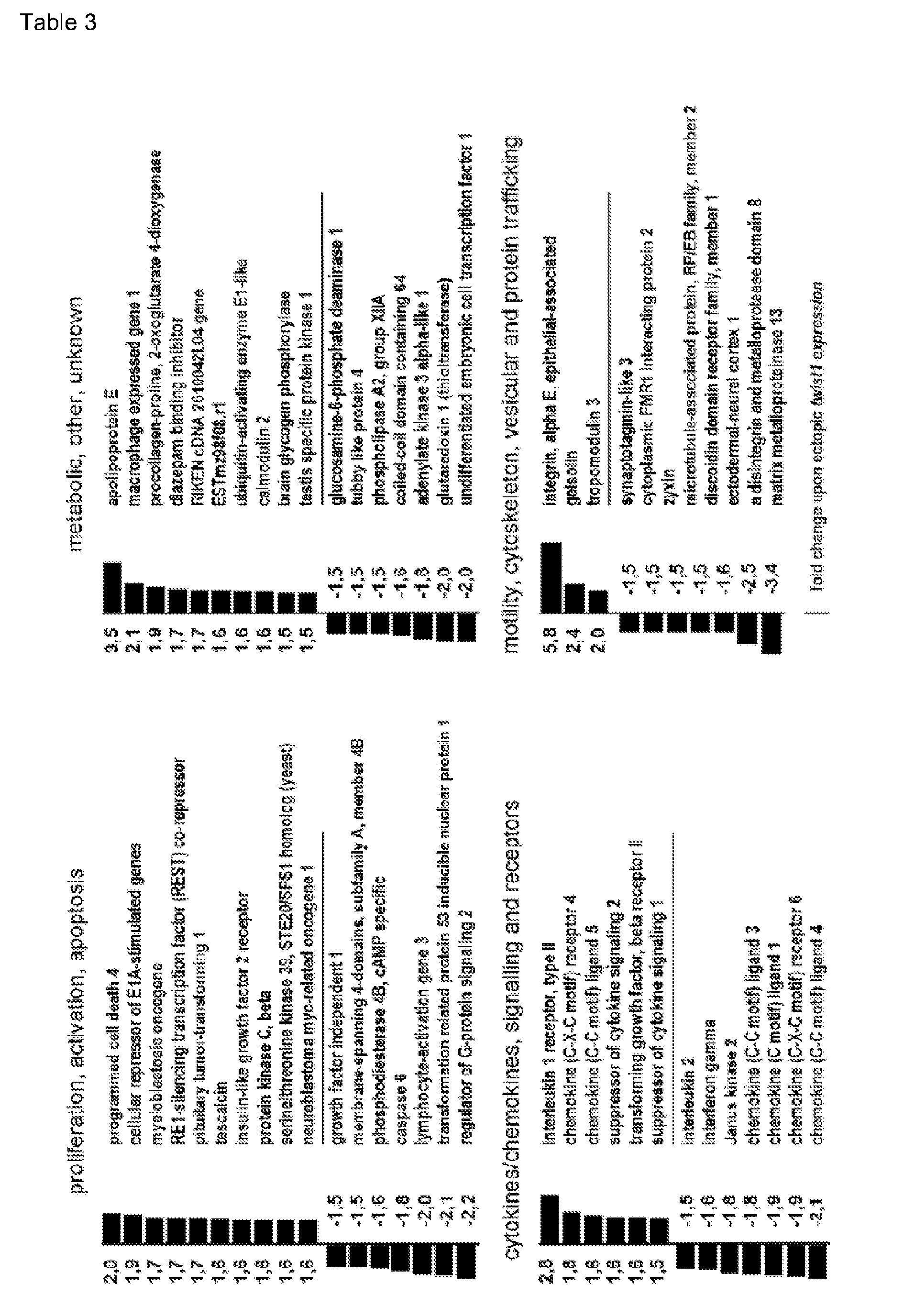

**Table S1**

| **Affymetr No** | **Gene symbol** | **Gene name** | **Fold change** | **t-test p-value** | **T_{H}1 1W** | **T_{H}1 4W** |
|---|---|---|---|---|---|---|
| 97994 at | Tc7 | transcription factor 7, T-cell specific | -50,7 | 6,47485E-09 | 1432 | 35 |
| 104578 f at | 3110023F 10Rik | RIKEN cDNA 3110023F 10 gene | -11,9 | 7,10526E-09 | 364 | 32 |
| 102282_g_at | Tnfrsf7 | tumor necrosis factor receptor superfamily, | -7,2 | 3,15546E-05 | 772 | 101 |
| 160667_at | Evl | Ena-vasodilator stimulated phosphoprotain | -6,6 | 3,20332E-07 | 289 | 41 |
| 96672_at | 2300002F06Rik | RIKEN cDNA 2300002F06 gene | 3.4 | 0.000107159 | 156 | 698 |
| 953333_at | II18rap | interleukin 18 receptor accessory protein | 3,4 | 9,00834E-06 | 81 | 287 |
| 102914_s at | Bcl2a1b | B-cell leukemia/lymphoma 2 related protein A1b | 3.5 | 2,70088E-07 | 190 | 628 |
| 97885_at | 1810009M01Rik | RIKEN cDNA 1810009M01 gene | 4,2 | 1,42213E-D7 | 301 | 1422 |
| 97949_at | Fgl2 | fibrinogen-like protein 2 | 4,4 | 9,86764E-07 | 64 | 307 |
| 96605 at | 0610011I04Rik | RIKEN cDNA 0610011I04 gene | 4.7 | 9,8031E-07 140 | 140 | 724 |
| 99370 at | Kirc1 | killer cell lectin-like receptor subfamily C, | 7,5 | 4,1522E-07 | 33 | 366 |
| 103024 at | Adam8 | a disintegrin and metalloprotease domain 8 | 9.9 | 7,20938E-06 | 48 | 672 |
| 99051_at | S100A4 | S100 calcium binding protein A4 | 10,5 | 1,27979E-06 | 176 | 1595 |
| 97519_at | Spp1 | secreted phosphoprotein 1 | 12.9 | 1.84635E-06 | 31 | 412 |
| 99957_at | Mmp9 | matrix metalloproteinase 9 | 19,0 | 9,75999E-07 | 12 | 219 |
| 98028_at | Twist1 | twist gene homolog, (Drosophila) | 38.8 | 7,14961E-05 | 7 | 225 |

**Table S2**

| **Patient ID** | **Relative *twist1* mRNA** | **Sex (male/female)** | **Age (years)** | **Sampling Date (m/y)** | **Disease duration (years)** | **ESR** | **CRP (mg/l)** |
|---|---|---|---|---|---|---|---|
| **Endoscopic biopsies from ulcerative colitis (UC) patients** | | | | | | | |
| UC1 | 414,8 | m | 48 | 01/04 | 3 | 103 | 0,4 |
| UC12 | 195,7 | | | 04/04 | | 88 | 0,3 |
| UC2 | 47,6 | m | 30 | 05/04 | 9 | 63 | 1,7 |
| UC3 | 32,8 | f | 38 | 11/04 | 8 | 94 | 9,13 |
| UC4 | 27,3 | f | 33 | 12/04 | 6 | NA | 19,6 |
| UC5 | 2,7 | m | 26 | 12/04 | <1 | NA | 49,8 |
| UC6 | 83,0 | m | 42 | 7/05 | 6 | NA | 3,5 |
| UC7 | 47,4 | m | 44 | 8/05 | 5 | NA | 14,9 |
| VC8 | 40,7 | m | 18 | 11/05 | 2 | 30 | 10 |
| UC9 | 10,9 | m | 67 | 12/05 | 15 | 88 | 25 |

| **Endoscopic biopsies from Crohn's disease (CD) patients** | | | | | | | |
|---|---|---|---|---|---|---|---|
| CD1 | 72,9 | m | 25 | 12/04 | 4 | NA | 43 |
| CD2 | 27,9 | f | 45 | 2/05 | 11 | NA | NA |
| CD3 | 23,8 | f | 41 | 3/05 | <1 | NA | 114,4 |
| CD4 | 31,8 | f | 22 | 7/05 | 3 | NA | 31,3 |
| CD5 | 12,7 | f | 34 | 8/05 | 5 | NA | 53,4 |
| CD6 | 13,3 | f | 21 | 11/05 | 5 | 27 | 10 |
| CD7 | 27,8 | f | 39 | 12/05 | 10 | 95 | 18 |

| **Synovial fluid from rheumatoid arthritis (RA) patients** | | | | | | | |
|---|---|---|---|---|---|---|---|
| RA1 | 30,8 | m | 68 | 03/04 | 1 | NA | 39,3 |
| RA2 | 12,5 | f | 40 | 02/05 | 13 | 10 | 12,3 |
| RA2 | 10,1 | | | 06/05 | | 12 | 6 |
| RA3 | 6,0 | f | 51 | 04/05 | 11 | 70 | 39 |
| RA4 | 15,9 | m | 68 | 08/05 | 1 | 30 | 14 |
| RA4 | 25,5 | | | 11/05 | | NA | NA |

| **Synovial fluid from reactive arthritis (ReA) patients** | | | | | | | |
|---|---|---|---|---|---|---|---|
| ReA1 | 30,0 | m | 28 | 03/04 | 1 | 25 | 35 |
| ReA2 | 7,4 | m | 43 | 06/05 | <1 | 50 | 116,4 |
| ReA3 | 9,2 | m | 31 | 11/05 | 1 | 10 | 0,3 |

| **Synovial fluid of psoriatic arthritis (PsA) patients** | | | | | | | |
|---|---|---|---|---|---|---|---|
| PsA1 | 54,9 | m | 55 | 03/04 | 10 | 9/22 | 0,3 |
| PsA2 | 14,2 | m | 68 | 02/05 | 2 | 39 | 34 |
| PsA3 | 32,7 | m | 50 | 07/05 | 19 | NA | NA |
| PsA3 | 24,1 | | | 09/05 | | NA | NA |

| **Synovial fluid from ankylosing spotidylitis (AS) patients** | | | | | | | |
|---|---|---|---|---|---|---|---|
| AS1 | 296,1 | m | 38 | 03/04 | 18 | 42 | 49 |
| AS1 | 171,1 | | | 08/04 | | 20 | 3 |
| AS1 | 130,1 | | | 09/04 | | NA | NA |
| AS1 | 168,3 | | | 03/05 | | 30 | 14,6 |
| AS1 | 138,6 | | | 09/05 | | 20 | 3,1 |
| AS2 | 13,6 | m | 73 | 07/04 | 8 | 65 | NA |
| AS2 | 9,4 | | | 12/04 | | NA | NA |
| AS3 | 2,4 | m | 20 | 12/04 | 5 | 70 | 86 |
| AS4 | 61,3 | m | 27 | 02/05 | 18 | NA | NA |
| AS5 | 5,5 | m | 21 | 10/05 | 1,5 | 80 | 4,5 |

| **Not included in** **figure 5b** | | | | | | | |
|---|---|---|---|---|---|---|---|
| Crohn's disease | 15,5 | NA | NA | 08/04 | surgical specimen | | |
| Crohn's disease | 45,9 | NA | NA | 11/05 | surgical specimen | | |
| Rheumatoid arthritis | 40,8 | NA | NA | 08/04 | NA | NA | NA |
| Rheumatoid arthritis | 14,8 | NA | NA | 09/04 | NA | NA | NA |
| undifferentiated SpA | 37,9 | f | 37 | 02105 | 1,5 | 20 | 2 |
| undiff. SpA | 12,8 | m | 44 | 09/06 | NA | NA | NA |
| undiff. arthritis | 3,0 | m | 48 | 8/05 | 1 | 18 | 6 |
| Lyme arthritis | 15,4 | m | 37 | 02/05 | <1 | 38 | 7 |
| Lyme arthritis | 3,5 | m | 21 | 06/05 | 2 | NA | NA |
| juvenile RA | 42,8 | f | 20 | 11/05 | 6 | 18 1,3 | 1,3 |

**Table S3**

| **Affymetr_No** | **Gene symbol** | **Gene name** | **Fold change** | **t-test p-value** | **Vector** | ***Twist1*** |
|---|---|---|---|---|---|---|
| 1449216_at | Itgae | integrin, alpha E, epithelial-associated | ***5,8*** | 0,003675582 | 45 | 257 |
| 1432466_a_at | Apoe | apolipoprotein E | ***3,5*** | 0,000333012 | 173 | 425 |
| 1419532 at | II1r2 | interleukin 1 receptor, type II | ***2,8*** | 1,90721E-13 | 784 | 2192 |
| 1415812 at | Gsn | gelsolin | ***2,4*** | 0,000427877 | 541 | 1282 |
| 1427076 at | Moeg1 | macrophage expressed gene 1 | ***2,1*** | 0,027175171 | 168 | 377 |
| 1456393 at | Pdcd4 | programmed cell death 4 | ***2,0*** | 0,000960162 | 800 | 1573 |
| 1423089 at | Tmod3 | tropomodulin 3 | ***2,0*** | 2,63289E-06 | 422 | 875 |
| 1426519 at | P4ha1 | procollagen-proline, 2-oxoglutarate 4-dioxygenase | ***1,9*** | 0,030113345 | 233 | 445 |
| 1415947 at | Creg | cellular repressor of E1A-stimulated genes | ***1,9*** | 2,00686E-06 | 832 | 1571 |
| 1448710 at | Cxcr4 | chemokine (C-X-C motif) receptor 4 | ***1,8*** | 0,0293381152 | 237 | 529 |
| 1455976 x at | Dbi | diazepam binding inhibitor | ***1,7*** | 0,001908118 | 1340 | 2276 |
| 1450194_a_at | Myb | myeloblastosis oncogene | ***1,7*** | 0,006922509 | 221 | 443 |
| 1417302_at | Rcor | RE1-silencing transcription factor co-repressor | ***1,7*** | 0,013680996 | 173 | 384 |
| 1428301_at | 2610042L04Rik | RIKEN cDNA 2610042L04 gene | ***1,7*** | 4,57606E-05 | 394 | 707 |
| 1438390_s_at | Pttg1 | pituitary tumor-transforming 1 | ***1,7*** | 0,001249245 | 625 | 1044 |
| 1418126_at | Ccl5 | chemokine (C-C motif) ligand 5 | ***1,6*** | 0,014298725 | 937 | 1699 |
| 1448021 at | ESTmz98f08.r1 | ESTmz98f08.r1 | ***1,6*** | 0,015998701 | 806 | 1337 |
| 1418744 s at | Tesc | tescalcin | ***1,6*** | 6,11991E-05 | 841 | 1507 |
| 1424112 at | Iqf2r | insulin-like growth factor 2 receptor | ***1,6*** | 0,010457946 | 731 | 1132 |
| 1460419 a at | Prkcb | protein kinase C, beta | ***1,6*** | 0,006315009 | 487 | 815 |
| 1419550_a_at | Stk39 | serine/threonine kinase 39, STE20/SPS1 homolog | ***1,6*** | 0,002358995 | 321 | 528 |
| 1425923_at | Nmyc1 | neuroblastoma myc-related oncogene 1 | ***1,6*** | 0,003631455 | 7548 | 11408 |
| 1449109_at | Socs2 | suppressor of cytokine signaling 2 | ***1,6*** | 0,001896075 | 805 | 1319 |
| 1426397_at | Tgfbr2 | transforming growth factor, beta receptor II | ***1,6*** | 0,000741228 | 2286 | 3306 |
| 1426970_a_at | Ube1I | ubiquitin-activating enzyme E1-like | ***1,6*** | 0,030406184 | 297 | 511 |
| 1422414_a_at | Calm2 | calmodulin 2 | ***1,6*** | 1,80435E-08 | 1587 | 2338 |
| 1433504_at | Pvgb | brain glycogen phosphorylase | ***1,5*** | 0,00183523 | 514 | 757 |
| 1450662 at | Tesk1 | tests specific protein kinase 1 | ***1,5*** | 0,000560519 | 766 | 1187 |
| 1450446 a at | Socs1 | suppressor of cytokine signaling 1 | ***1,5*** | 0,000193353 | 936 | 1366 |
| 1455065 x at | Gnpda1 | qluoosamine-6-phosphate deaminase 1 | ***-1,5*** | 0,00169187 | 1430 | 956 |
| 1448548 at | Tulp4 | tubby like protein 4 | ***-1,5*** | 2,19399E-05 | 1144 | 820 |
| 1452026_a_at | Pla2g12a | phospholipase A2, group XIIA | ***-1,5*** | 0,013979562 | 1228 | 826 |
| 1449990_at | II2 | interleukin 2 | ***-1,5*** | 0,000138665 | 8582 | 5654 |
| 1449273 at | Cyfip2 | cytoplasmic FMR1 interacting protein 2 | ***-1,5*** | 0,000719924 | 1325 | 983 |
| 1417240_at | Zyx | zyxin | ***-1,5*** | 0,000445837 | 876 | 601 |
| 1425787_a_at | Syti3 | synaptotagmin-like 3 | ***-1,5*** | 6,82725E-06 | 2239 | 1478 |
| 1426245 s_at | Mapre2 | microtubule-associated protein, RP/EB family, 2 | ***-1,5*** | 0,022816609 | 1224 | 791 |
| 1417679_at | Gfi1 | growth factor independent 1 | ***-1,5*** | 8,3341E-05 | 2506 | 1619 |
| 1423467 at | Ms4a4b | membrane-spanning 4-domains A, member 4B | ***-1,5*** | 0,001242527 | 4717 | 3005 |
| 1456226_x_at | Ddr1 | discoidin domain receptor family, member 1 | ***-1,6*** | 0,004473965 | 593 | 367 |
| 1425947 at | Ifng | interferon gamma | ***-1,6*** | 0,018605196 | 6646 | 4323 |
| 1426816 at | Ccdc64 | coiled-coil domain containing 64 | ***-1,6*** | 0,000603641 | 705 | 438 |
| 1422473 at | Pde4b | phosphodiesterase 4B, cAMP specific | ***-1,6*** | 1,66039E-05 | 1133 | 627 |
| 1420965_a_at | Enc1 | ectodermal-neural cortex 1 | ***-1,7*** | 0,001533107 | 1510 | 894 |
| 1421065_at | Jak2 | Janus kinase 2 | ***-1,8*** | 1,41758E-07 | 4700 | 2734 |
| 1419561 at | Ccl3 | chemokine (C-C motif) ligand 3 | ***-1,8*** | 0,03228739 | 3034 | 1616 |
| 1450387_s_at | Ak3I1 | adenylate kinase 3 alpha-like 1 | ***-1,8*** | 5,09427E-06 | 744 | 427 |
| 1415995 at | Casp6 | caspase 6 | ***1,8*** | 0,000232808 | 1137 | 573 |
| 1419412_at | Xcl1 | chemokine (C motif) ligand 1 | ***-1,9*** | 0,001140306 | 2274 | 1208 |
| 142281_at | Cxcr6 | chemokine (C-X-C motif) receptor 6 | ***-1,9*** | 0,000118139 | 739 | 375 |
| 1416593 at | Glrx1 | glutaredoxin 1 (thioltransferase) | ***-2,0*** | 0,000130173 | 853 | 444 |
| 1449911_at | Lag3 | lymphocyte-activation gene 3 | ***-2,0*** | 0,000660697 | 1455 | 638 |
| 1416899 at | Utf1 | undifferentiated embryonic cell transcription factor 1 | ***-2,0*** | 0,000102794 | 663 | 378 |
| 1416926 at | Trp53inp1 | transformation related p53 inducible nuclear protein 1 | ***-2,1*** | 0,00167533 | 2514 | 1087 |
| 1421578 at | Ccl4 | chemokine (C-C motif) ligand 4 | ***-2,1*** | 0,000600675 | 7169 | 3053 |
| 1419247_at | Rgs2 | regulator of G-protein signaling 2 | ***-2,2*** | 8,12651E-06 | 830 | 385 |
| 1416871_at | Adam8 | a disintegrin and metailoprotease domain 8 | ***-2,5*** | 0,000112885 | 716 | 281 |
| 1417256_at | Mmp13 | matrix metailoproteinase 13 | ***-3,4*** | 1,70802E-06 | 689 | 190 |

**Table S4**

| | **Acute inflammation** | | | | **Chronic inflammation** | | | | ***sum*** | |
|---|---|---|---|---|---|---|---|---|---|---|
| **mouse** | **exudate** | **gran. inf. SM** | **fibrin** | **periart. gran.** | **hyperpl.** | **mono. inf SM** | **peri. mono.** | **cartilage destr.** | ***acute*** | ***chronic*** |
| scrT 1 | 0 | 1 | 0 | 0 | 0 | 0.5 | 0 | 0 | 1 | 0.5 |
| scrT 2 | 0 | 1 | 0 | 0 | 1 | 1 | 2 | 0 | 1 | 4 |
| scrT 3 | 0 | 1 | 1 | 0 | 0 | 0.5 | 0 | 0 | 2 | 0.5 |
| scrT 4 | 1 | 1 | 1 | 0 | 0 | 1 | 0 | 0 | 3 | 1 |
| scrT 5 | 0 | 1 | 1 | 0 | 0 | 1 | 0 | 03 | 2 | 1 |
| Twist 1 | 1 | 1 | 1 | 1 | 0 | 1.5 | 1 | 0 | 4 | 2.5 |
| Twist 2 | 1 | 2 | 1 | 1 | 1 | 2 | 1 | 0 | 5 | 5 |
| Twist 3 | 1 | 2 | 1 | 1 | 1 | 1.5 | 1 | 0 | 5 | 3.5 |
| Twist 4 | 2 | 3 | 1 | 1 | 1 | 2 | 1 | 1 | 7 | 4 |
| Twist 5 | 2 | 3 | 1 | 1 | 0 | 2 | 2 | 1 | 7 | 4 |

**Table S5**

| **Primer sequences:** | |
|---|---|
| | **Construction of shRNA-expression retrovirus:** |
| Xbal EF1aGFP forward | TTCTAGAGACGATAAGCTTTGCAAAGATG |
| EcoR5 EF1aGFP reverse | TGATATCCATATGACTAGTCCCCGAAGTTG |
| shTwist1 forward | TGCTGAGCAAGATTCAGACCTTCAAGAGAGGTCTGAATCTTGCTCAGCTTTTTTC |
| shTwist1 reverse | TCGAGAAAAAAGCTGAGCAAGATTCAGACCTCTCTTGAAGGTCTGAATCTTGCTCAGCA |
| scr shTwist1 forward | TGCTATCGAGAAGATCAGCCTTCAAGAGAGGCTGATCTTCTCGATAGCTTTTTTC |
| scr shTwist1 reverse | TCGAGAAAAAAGCTATCGAGAAGATCAGCCTCTCTTGAAGGCTGATCTTCTCGATAGCA |
| Xhol U6shRNA forward | TATCTCGAGCAGAGATCCAGTTTGGTTAGTACC |
| U6shRNA reverse | TAGGTCCCTCGACCTGCTGG |
| | |

| | **Construction of protein-expression retrovirus:** |
|---|---|
| BgIII mTbet forward | ATGGAAGATCTATGGGCATCGTGGAGCC |
| Xhol mTbet reverse | ATCCGCTCGAGTCAGTTGGGAAAATAATTATAAAAC |
| BgIII mTwist1 forward | GAAGATCTATGATGCAGGACGTGTCCAGC |
| Xhol mTwist reverse | ATCCGCTCGAGCTAGTGGGACGCGGACATGG |
| BgIII hMIkBa forward | ATGGAAGATCTATGTTCCAGGCGGCCGA |
| SaII hMIkBa reverse | TTCGTCGACTCATAACGTCAGACGCTGGC |
| | |

| | **Real-time PCR** |
|---|---|
| Murine HPRT forward | GCTGGTGAAAAGGACCTCT |
| Murine HPRT reverse | CACAGGACTAGAACACCTGC |
| Murine Foxp3 forward | CTGCTCCTCCTATTCCCGTAAC |
| Murine Foxp3 reverse | AGCTAGAGGCTTTGCCTTCG |
| Murine Twist2 forward | GCATCCTGGCCAACGTGC |
| Murine Twist2 reverse | TCCATGCGCCACACGGAG |
| Murine Twist1 forward | CGCACGCAGTCGCTGAACG |
| murine/hum Twist1 reverse | GACGCGGACATGGACCAGG |
| Human Twist1 forward | GGCACCCAGTCGCTGAACG |
| Human IL4 forward | CGGCAGTTCTACAGCCACCATG |
| Human IL4 reverse | CCAACGTACTCTGGTTGGCTTC |
| Human DATA-3 forward | GAACCGGCCCCTCATTAAG |
| Human GATA-3 reverse | ATTTTTCGGTTTCTGGTCTGGAT |
| Human T-bet forward | CCCCGGCTGCATATCG |
| Human T-bet reverse | ATCCTTTGGCAAAGGGGTTA |
| Human UbcH5 forward | TCTTGACAATTCATTTCCCAACAG |
| Human UbcH5 reverse | TCAGGCACTAAAGGATCATCTGG |
| Human Foxp3 forward | TTTCACCTACGCCACGCTCATCC |
| Human Foxp3 reverse | CTCTCCACCCGCACAAAGCACTT |
| Human IFN-γ forward | CGAGATGACTTCGAAAAGCTG |
| Human IFN-γ reverse | ATATTGCAGGCAGGACAACC |

## Claims

1. A method for in-vitro detecting pathogenic T helper cells comprising the steps of:
- incubating a sample of a body fluid or tissue taken from a mammal with substances specifically interacting with at least one gene product encoded by a gene that is selected from the group comprising the genes of Table 1 and Table 2,
- determining specific incubation products,
- correlating an amount of signal or change in signal with a concentration of the gene product in the sample, and
- detecting cell pathogenicity by comparing the concentration with another gene product concentration in a sample of non-pathogenic and/or pathogenic cells.

2. Use of substances specifically interacting with at least one gene product encoded by a gene that is selected from the group comprising the genes of Table 1 and Table 2 for detecting pathogenic T helper cells in-vitro.

3. Kit for use in detection of pathogenic T helper cells comprising substances specifically interacting with at least one gene product encoded by a gene that is selected from the group comprising the genes of Table 1 and Table 2.

4. Pharmaceutical composition comprising as active ingredient an effective amount of at least one protein encoded by a gene that is selected from the group comprising the genes of Table 1 and Table 2, optionally together with pharmaceutically tolerable adjuvants.

5. Pharmaceutical composition comprising as active ingredient an effective amount of at least one substance specifically interacting with at least one gene that is selected from the group comprising the genes of Table 1 and Table 2, or a regulator protein or a gene product thereof, or a component of a signal transduction pathway comprising said gene or a gene product thereof, optionally together with pharmaceutically tolerable adjuvants.

6. A protein, which is encoded by a gene that is selected from the group comprising the genes of Table 1 and Table 2, for the diagnosis, prophylactic or therapeutic treatment and/or monitoring of chronic inflammatory diseases.

7. The protein according to claim 6, which is encoded by a gene that is selected from the group comprising the genes of Table 1, preferably Twist1, for the diagnosis, prophylactic or therapeutic treatment and/or monitoring of chronic inflammatory diseases, preferably spondyloarthopathies, arthrosis, rheumatic diseases, rheumatoid arthritis, juvenile idiopathic arthritis, Crohn's disease, ulcerative colitis, diabetes, inflammatory bowel disease, Multiple Sclerosis and/or Systemic Lupus Erythematosus.

8. The protein according to claim 6, which is encoded by a gene that is selected from the group comprising the genes of Table 2, for the diagnosis, prophylactic or therapeutic treatment and/or monitoring of allergic diseases, preferably allergic inflammations.

9. A substance, which specifically interacts with at least one gene that is selected from the group comprising the genes of Table 1 and Table 2, or a regulator protein or a gene product thereof, or a component of a signal transduction pathway comprising said gene or a gene product thereof, for the diagnosis, prophylactic or therapeutic treatment and/or monitoring of chronic inflammatory diseases.

10. A method for screening substances, which reduce pathogenicity of T helper cells, comprising the steps of:
- providing a sample of pathogenic T helper cells, which are capable of differentially expressing a gene that is selected from the group comprising the genes of Table 1 and Table 2, in comparison with non-pathogenic T helper cells,
- dividing the sample into portions,
- incubating at least one portion with substances to be screened,
- comparing the expression pattern and/or cell viability in the portion with another portion that is not incubated with the substances, and
- detecting the specific binding of substances to said gene or a regulatory associated gene or a regulator protein or a gene product thereof, or a component of a signal transduction pathway comprising said gene or a regulatory associated gene or a gene product thereof.

11. A method for treating chronic inflammatory diseases, wherein an effective amount of at least one substance specifically interacting with at least one gene that is selected from the group comprising the genes of Table 1 and Table 2, or a regulator protein or a gene product thereof, or a component of a signal transduction pathway comprising said gene or gene products thereof, is administered to a mammal in need of such treatment.
